(19) Europäisches Patentamt
European Patent Office
Office européen des brevets

(11) EP 2 613 828 B1

(12) **EUROPEAN PATENT SPECIFICATION**

(45) Date of publication and mention
of the grant of the patent:
**27.11.2019 Bulletin 2019/48**

(51) Int Cl.:
***A61M 15/00*** *(2006.01)*

(21) Application number: **11749866.7**

(86) International application number:
**PCT/EP2011/065301**

(22) Date of filing: **05.09.2011**

(87) International publication number:
**WO 2012/032008 (15.03.2012 Gazette 2012/11)**

(54) **METERED-DOSE INHALER ACTUATOR, METERED-DOSE INHALER**

INHALIERGERÄTBETÄTIGUNG MIT ABGEMESSENER DOSIS, INHALIERGERÄT MIT ABGEMESSENER DOSIS

ACTIONNEUR D'INHALATEUR DE DOSE MESURÉE, INHALATEUR DE DOSE MESURÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.09.2010 EP 10175427**

(43) Date of publication of application:
**17.07.2013 Bulletin 2013/29**

(73) Proprietor: **CHIESI FARMACEUTICI S.p.A.**
**43122 Parma (IT)**

(72) Inventors:
• **BRAMBILLA, Gaetano**
**I-43122 Parma (IT)**
• **LEWIS, David Andrew**
**I-43122 Parma (IT)**
• **JOHNSON, Robert**
**I-43122 Parma (IT)**

(74) Representative: **Adam, Holger**
**Kraus & Weisert**
**Patentanwälte PartGmbB**
**Thomas-Wimmer-Ring 15**
**80539 München (DE)**

(56) References cited:
**GB-A- 2 104 393      US-A- 3 001 524**
**US-A- 4 796 614      US-A- 5 435 297**
**US-A- 5 669 376      US-A- 6 062 214**

**Description**

FIELD OF THE INVENTION

The invention relates to a metered-dose inhaler actuator, a metered-dose inhaler and a method of using the same.

BACKGROUND OF THE INVENTION

Among the devices available to deliver medicaments to the lung, metered-dose inhalers (MDIs) are widely used.

MDIs are aerosol delivery systems designed to deliver a medicament formulated with a solvent, such as a compressed, low boiling point liquid gas propellant. MDIs are designed to meter a predetermined quantity of the medicament, completely dissolved (in solution) or suspended in the formulation and dispense the dose as an inhalable aerosol cloud or plume.

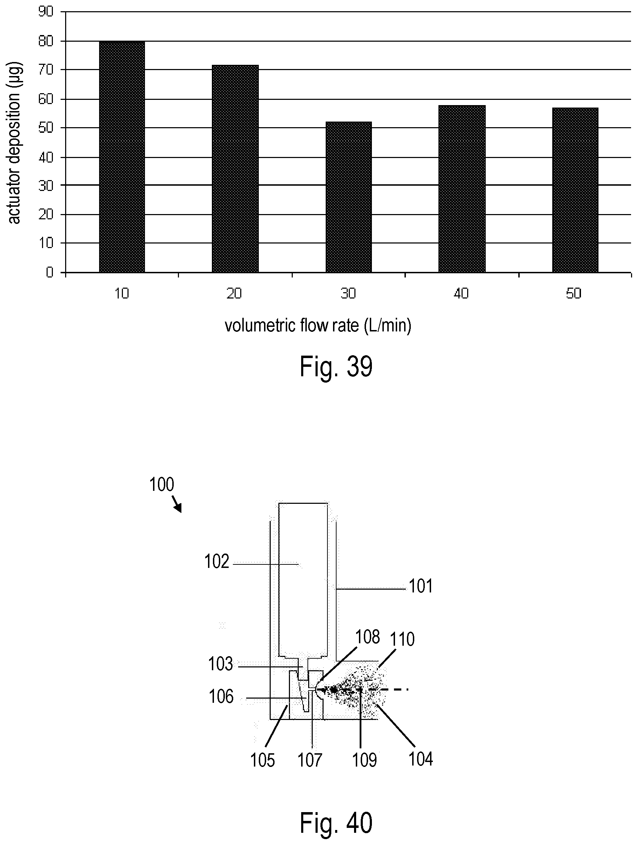

A conventional MDI 100 is shown in Fig. 40. The MDI 100 includes an actuator 101 in which a canister 102 is positioned. The canister 102 contains a formulation wherein the medicament is in solution or in suspension with a low boiling point propellant. The canister 102 is normally provided with a metering valve having a hollow valve stem 103 for measuring discrete doses of the medicament formulation. The dose is dispensed as an inhalable cloud or plume 104.

Typical actuators 101 have a nozzle or valve stem block 105 which receives the hollow valve stem 103 of the aerosol canister 102. The valve stem block 105 defines the walls of the valve stem receptacle, expansion chamber 106, and orifice 107. The orifice 107 serves to propel the aerosol formulation towards a mouthpiece opening 110 and assists in atomization of the aerosol formulation. Traditionally, the orifice 107 has been provided such that its longitudinal axis is aligned with a longitudinal axis 109 of the actuator mouthpiece portion, so that the aerosol exits the orifice in a mean direction towards a mouthpiece opening 110. I.e., the orifice 107 in the stem block 105 has traditionally been located at an angle from approximately 90° to approximately 110° to the direction of the hollow valve stem 103, such that when the canister 102 is actuated, formulation containing propellant moves down the stem 103 and expands within the expansion chamber 106 before being propelled through the orifice 107 towards the mouthpiece opening 110. The formulation is atomised in a direction extending at an angle from approximately 90° to approximately 110° from a longitudinal direction of the aerosol canister 102. Examples for an arrangement of the valve stem block 105 in an actuator housing as illustrated in Fig. 40 are described, for example, in WO 2009/003657 A1.

In the traditional actuator design as shown in Fig. 40, the manufacturing process imposes constraints on the possible shapes of the orifice 107 which can be realized in the valve stem block 105. For illustration, in traditional molding procedures, a pin may be provided in a mold so as to allow the orifice 107 to be formed. As the pin needs to be withdrawn from the opening after the actuator has been molded, orifice designs may be limited to cylindrical shapes or to shapes which flare towards the mouthpiece opening 110. For illustration, a flared portion 108 may be formed on an exterior face of the valve stem block 105 and around the exit opening of the orifice 107.

Due to the orientation of the orifice 107 and the expansion chamber 106 within the stem block 105, modifications to orifice design are limited. For illustration, some modifications may be made to explore the effect of various orifice diameters and orifice lengths for cylindrical orifices 107. However, greater flexibility in orifice design would be desirable.

While attaining greater flexibility in orifice design is desirable, the actuator performance should at least be comparable, or even superior, to traditional designs with regard to certain characteristics. For illustration, it may be desirable to have greater flexibility in orifice design while reducing the proportion of non-respirable particles or droplets which are dispensed from the actuator in an inhalation process.

The influence of airflow patterns on actuator characteristics has been addressed in various contexts in the art. For illustration, US 4,972,830 describes an inhaler in which the passage which directs the pressurized medicament from the canister to a mouthpiece opening has a particular configuration to reduce the velocity of the spray and enhance dispersion of the medicament in the airflow. The inhaler of US 4,972,830 has a conventional arrangement of the orifice, oriented at an angle of 90° relative to the valve stem axis, which makes it challenging to use orifice shapes tapering toward the mouthpiece opening in conventional mass production techniques.

US 5,435,297 describes an inhaler for metered aerosol having a cylindrical housing with a chamber to receive an aerosol container, main air channels extending axially within the housing and a mouthpiece coaxially connected to the housing. Further inhalation devices are described in US 3,001,524, in GB 2104393 A, in US 6,062,214, in US 5,669,376, and in US 4,796,614.

In view of the above, there is a continued need in the art for actuators for metered-dose inhalers and for metered-dose inhalers which address some of the above needs. In particular, there is a continued need for actuators for metered-dose inhalers and for metered-dose inhalers which allow a greater variety of orifice shapes to be realized. There is also a need for such actuators and metered-dose inhalers which allow a substantial fraction of non-respirable particles or droplets to be removed from an aerosol cloud before the aerosol cloud is dispensed through a mouthpiece opening.

SUMMARY

These and other needs are addressed by a metered-dose inhaler actuator, a metered-dose inhaler and a method of using the same as defined in claims 1, 13 and 14. The dependent claims define embodiments.

According to an aspect, a metered-dose inhaler actuator is provided. The actuator comprises a housing having a mouthpiece portion and a canister receiving portion configured to receive a canister. The housing extends from an opening for receiving the medicament canister to a mouthpiece opening. The actuator further comprises a member disposed within the housing and defining a valve stem receptacle configured to receive a valve stem of the canister. An orifice is formed in the member, which orifice is in fluid communication with the valve stem receptacle and extends to a face of the member opposite to the valve stem receptacle. A longitudinal axis of the orifice is aligned with a longitudinal axis of the valve stem receptacle. At least one air inlet opening is provided in an outer shell of the housing in spaced relation from the opening for receiving the medicament canister and the mouthpiece opening, the at least one air inlet opening being in fluid communication with the mouthpiece opening.

As used herein, the term "aligned" when referring to two axes means "coinciding or parallel to each other".

In the actuator, the longitudinal axis of the orifice is aligned with the longitudinal axis of the valve stem receptacle. This allows a greater variety of orifice shapes to be realized even when using conventional actuator manufacturing techniques. The orientation of the longitudinal axis of the orifice allows a greater variety of orifice shapes to be realized without requiring a member defining the orifice to be produced separately from the housing of the actuator. Non-respirable particles or droplets may impact on an inner surface of the actuator housing, so that a significant fraction of the non-respirable particles or droplets may be removed prior to the aerosol cloud or plume being dispensed from the actuator. For illustration, an orifice having a tapering portion may be formed, the portion tapering in a direction away from the valve stem receptacle. The at least one air inlet opening provided in the outer shell of the housing allows an airflow to be established in the housing which entrains the particles or droplets, when the actuator is put into use.

The actuator is designed such that atomized spray may be emitted from the orifice with a longitudinal axis which coincides with the longitudinal axis of the valve stem receptacle and, in use of the device, with a longitudinal axis of the canister.

The at least one air inlet opening may be provided in a part of the outer shell of the housing which extends from the member defining the valve stem receptacle towards the mouthpiece opening. Thereby, an airflow may be established which allows a high fine particle fraction to be delivered.

The mouthpiece portion may have a longitudinal axis and the housing may have a wall which is oriented at an angle relative to the longitudinal axis of the mouthpiece portion (i.e., which is not parallel to the longitudinal axis of the mouthpiece portion). An air inlet opening of the at least one air inlet opening may be provided in the wall. The wall may extend essentially parallel to the longitudinal axis of the orifice. The wall may be a rear wall of the canister receiving portion. Thereby, an airflow may be established which allows a high fine particle fraction to be delivered.

An air inlet opening may be positioned such that it is visible through the mouthpiece opening for at least one viewing direction. All air inlet openings may be positioned such that they are visible through the mouthpiece opening for at least one viewing direction. Thereby, an airflow pattern can be established, in use of the actuator, in which the airflow interacts with the aerosol plume. Respirable particles or droplets can be efficiently transported towards the mouthpiece opening in the airflow pattern.

An air inlet opening may be positioned in a base of the actuator, which is defined by a boundary of the mouthpiece portion which, in operation of the actuator, is the lower boundary of the mouthpiece portion. Plural air inlet openings may be positioned in the base of the actuator. By positioning one or plural air inlet openings on the base of the actuator, an air flow is produced which, in proximity to the air inlet openings, has a direction almost opposite to the direction of the plume. Actuator deposition may thereby be reduced. This may improve the aerosol performance. A high fine particle fraction may be attained. For air inlet opening(s) positioned on the actuator base, the distance between the orifice and the air inlet opening(s) may be larger than for air inlet opening(s) positioned in a side wall of the actuator. The number and position of the air inlet opening(s) may be selected as a function of a distance between the orifice and the actuator base.

At least one of the air inlet opening(s) formed in the actuator base may be positioned towards the rear wall of the actuator, relative to an impaction point of the plume. I.e., the intersection point of the longitudinal axis of the orifice with the actuator base may have a distance from the mouthpiece opening which is smaller than a distance of the at least one air inlet opening in the base from the mouthpiece opening, the distance being respectively measured along a line parallel to the longitudinal axis of the mouthpiece portion.

If more than one air inlet opening is positioned in the actuator base, an offset between the air inlet openings in a direction transverse to the longitudinal axis of the mouthpiece portion may be set so as to correspond to a width of the plume as it impacts onto the actuator base.

Additionally or alternatively, several air inlet openings may be positioned in the actuator base around the intersection point of the longitudinal axis of the orifice with the actuator base.

An air inlet opening may be positioned on a straight line which is parallel to a longitudinal axis of the mouthpiece portion and which passes through the mouthpiece opening. The actuator may be configured such that the straight line passes through a hollow interior of the housing, without passing through any solid actuator components. This allows an airflow pattern to be established, in use of the actuator, in which respirable particles or droplets can be efficiently transported towards the mouthpiece opening.

The member and the air inlet opening may be configured such that, in use of the actuator, all air output via the mouthpiece opening is drawn into an interior of the housing through the at least one air inlet opening. This allows airflow patterns in the housing to be controlled via the position of the at least one air inlet opening.

The member may extend across a cross section area of the canister receiving portion. This allows the member to provide adequate support for a canister in use of the actuator, while an arrangement with the longitudinal axes of the orifice and the valve stem receptacle being aligned with each other can be implemented in a simple geometry.

The member may be configured to block passage of gas past the member radially outwardly of the orifice. I.e., the member may be configured such that gas may exit from the face which is opposite to the valve stem receptacle only through the orifice. In use of the actuator, air flows directed along the longitudinal axis of the canister receiving portion and head on towards an actuator base may be reduced or prohibited.

The mouthpiece portion may define a base of the actuator, and the member may be disposed spaced from the base. The member may in particular be disposed in the canister receiving portion, so that it is not visible through the mouthpiece opening. Thereby, an impact of the member on the airflow pattern from the at least one air inlet opening to the mouthpiece opening may be reduced or prohibited.

A distance between a plane of a face of the member in which the exit of the orifice is located and the base of the actuator, measured along a rear wall of the actuator, may define the base height. The base height may be in the range from 8 mm to 52 mm. The base height may in particular be in the range from 12 mm to 32 mm. The base height may in particular be in the range from 12 mm to 22 mm. The base height may in particular be 22 mm. For such base heights, high fine particle doses can be attained.

The orifice may have at least a portion tapering towards the face of the member opposite to the receptacle. Thereby, atomization of aerosol formulations containing a high concentration of polar low volatile compounds, which may be one or more polar co-solvents such as an alcohol, water or a glycol, may be improved.

A maximum diameter of the tapering portion of the orifice may be matched to an outer diameter of the valve stem. Thereby, deposition of drugs within the valve stem may be reduced.

A maximum diameter of the tapering portion of the orifice may be matched to an inner diameter of the valve stem. Thereby, formation of eddy currents directly below the valve stem may be reduced, and deposition of drugs within the valve stem may be reduced.

An expansion chamber may be formed in the member. The expansion chamber may be in fluid communication with the orifice and the valve stem receptacle and may have a longitudinal axis aligned with the longitudinal axis of the valve stem receptacle. Thereby, an internal expansion chamber may be integrated in an in-line configuration with the valve stem receptacle and the orifice, depending on the requirements imposed by the aerosol formulation to be delivered. The expansion chamber may have at least a portion tapering towards the face of the member opposite to the valve stem receptacle. The tapering portion of the expansion chamber may provide a smooth transition to the orifice.

A longitudinal axis of the orifice may be disposed at an angle equal to or greater than 90° relative to a longitudinal axis of the mouthpiece portion. This configuration may assist in allowing a greater amount of fine particles or droplets to be entrained in an airflow across an actuator base.

In any one of the embodiments, the longitudinal axis of the orifice may coincide with the longitudinal axis of the valve stem receptacle. If an expansion chamber is integrated in the member, a longitudinal axis of the expansion chamber may coincide with the longitudinal axis of the valve stem receptacle.

The actuator may be configured as an actuator for a breath actuated inhaler (BAI). This allows the actuator to be used in a system which eliminates the need for manual coordination by automatically actuating the release of a dose of aerosol when the patient inhales with his/her lips in contact with the mouthpiece.

When the actuator is configured as an actuator for a BAI, the actuator may be configured such that the air flow is initiated prior to the actuation of a valve assembly, i.e., prior to dispensing a dose from the canister. A good response may thereby be attained.

The actuator may include components to automatically actuate release of a dose from a medicament container when the patient inhales with his/her lips in contact with the mouthpiece. For a thus configured actuator, a single inspiration effort of the patient may deliver a dose of the aerosol and may drive the separation of respirable and non-respirable particles of the plume.

According to a further aspect, a metered-dose inhaler is provided. The metered-dose inhaler comprises the actuator of any one aspect or embodiment described herein, and a canister having a metering valve. The canister comprises a valve stem to be fitted into the valve stem receptacle formed in the member of the actuator. The canister contains an aerosol formulation.

The aerosol formulation may be an aerosol solution formulation or an aerosol suspension formulation. The aerosol formulation may contain at least one active ingredient in a propellant or in a propellant/solvent system and, optionally, further excipients.

The metered-dose inhaler may be a breath actuated inhaler. This configuration eliminates the need for manual coordination in use of the inhaler by automatically actuating the release of a dose of aerosol when the patient inhales with his/her lips in contact with the mouthpiece. Further, a single inspiration effort of the patient may deliver a dose of the aerosol and may drive the separation of respirable and non-respirable particles of the plume.

According to another aspect, a method is provided in which an actuator of any one aspect or embodiment described herein is used for dispensing an aerosol formulation from a canister without interaction with a human or animal body. The method may, for example, be used to dispense an aerosol formulation when priming a metered dose inhaler.

The aerosol formulation may be an aerosol solution formulation or an aerosol suspension formulation. The aerosol formulation may contain at least one active ingredient in a propellant or in a propellant/solvent system and, optionally, further excipients.

According to another aspect, a metered-dose inhaler actuator is provided. The actuator comprises a housing having a mouthpiece portion and a canister receiving portion configured to receive a canister. The actuator further comprises a member disposed within the housing and defining a valve stem receptacle configured to receive a valve stem of the canister. An orifice is formed in the member, which orifice is in fluid communication with the valve stem receptacle and extends to a face of the member opposite to the valve stem receptacle. The orifice formed in the member has a portion tapering towards the face of the member disposed opposite to the receptacle.

With the actuator according to the other aspect, atomization of aerosol formulations containing a high concentration of polar compounds can be improved.

In the actuator according to the other aspect, a longitudinal axis of the orifice may be aligned with a longitudinal axis of the valve stem receptacle. If an expansion chamber is formed in the member, a longitudinal axis of the expansion chamber may also be aligned with the longitudinal axis of the valve stem receptacle. This configuration allows the tapering portion to be readily formed upon manufacture of the actuator.

In the actuator according to the other aspect, at least one air inlet opening may be provided in an outer shell of the housing.

According to an example, a method of manufacturing a metered dose inhaler actuator includes forming a housing having a mouthpiece portion and a canister receiving portion configured to receive a canister, with the housing extending from an opening for receiving the medicament canister to a mouthpiece opening. The method includes forming a member disposed within the housing and defining a valve stem receptacle configured to receive a valve stem of the canister, wherein an orifice is formed in the member so that the orifice is in fluid communication with the valve stem receptacle and extends to a face of the member opposite to the valve stem receptacle. The member is formed such that a longitudinal axis of the orifice is aligned with a longitudinal axis of the valve stem receptacle. At least one air inlet opening is formed in an outer shell of the housing in spaced relation from the opening for receiving the medicament canister and the mouthpiece opening, the at least one air inlet opening being formed so as to be in fluid communication with the mouthpiece opening.

The member may be formed such that an exit opening of the orifice is located at a distance from a base of the actuator. A position of the at least one air inlet opening may be selected as a function of this distance. A count of air inlet openings comprised by the at least one air inlet opening may be selected as a function of the distance between the exit opening of the orifice and the base of the actuator.

Various effects may be attained with actuators, metered dose inhalers and methods of embodiments. For illustration, an actuator according to an embodiment may be designed so as to attain a reduced deposition of drug within the oro-pharyngeal region.

The above and other effects will be illustrated further with reference to exemplary embodiments described with reference to the drawings.

BRIEF DESCRIPTION OF THE DRAWINGS

Fig. 1 is a schematic cross-sectional view of a metered-dose inhaler including an actuator of an embodiment.

Fig. 2 is a schematic front view of the metered-dose inhaler of Fig. 1.

Fig. 3 is a schematic cross-sectional view of a metered-dose inhaler including an actuator of another embodiment.

Fig. 4 is a schematic cross-sectional view of a metered-dose inhaler including an actuator of another embodiment.

Fig. 5 is a schematic cross-sectional view of a metered-dose inhaler including an actuator of another embodiment.

Fig. 6 is a diagram representing a delivered dose for various actuator designs.

Fig. 7 is a diagram illustrating an exterior configuration of a metered-dose inhaler having an actuator according to an embodiment (on the right) compared to a cross-sectional view of a conventional metered-dose inhaler (on the left).

Fig. 8 is a diagram representing a delivered dose for various actuator designs.

Figs. 9-14 illustrate orifice designs in actuators according to embodiments.

Fig. 15 is a schematic cross-sectional view of a metered-dose inhaler including an actuator of another embodiment.

Fig. 16 is a schematic view illustrating an actuator base of an actuator according to another embodiment.

Fig. 17 is a schematic view illustrating various configurations of air inlet openings.

Fig. 18A and 18B are schematic views illustrating configurations of air inlet openings positioned on an actuator rear wall and an actuator base, respectively.

Fig. 19 is a schematic diagram of an apparatus used to measure a pressure drop.

Figs. 20A, 20B and 20C are diagrams representing delivery characteristics of actuators according to various embodiments having air inlet openings located in an actuator base, for three different formulations.

Figs. 21A, 21B and 21C are diagrams representing delivery characteristics of actuators according to various embodiments having air inlet openings located in a rear wall of the actuator, for three different formulations.

Fig. 22A is a diagram illustrating a pressure drop for actuators according to various embodiments having air inlet openings located in an actuator base, and Fig. 22B is a diagram illustrating a pressure drop for actuators according to various embodiments having air inlet openings located in a rear wall of the actuator.

Fig. 23 is a diagram representing delivery characteristics of actuators according to various embodiments which have one air inlet opening located in an actuator base, for different diameters of the air inlet openings.

Fig. 24 is a diagram representing delivery characteristics of actuators according to various embodiments, for different arrangements and sizes of air inlet openings.

Fig. 25 is a diagram representing delivery characteristics of actuators according to various embodiments which have two air inlet openings located in an actuator base, for different diameters of the air inlet openings.

Fig. 26 is a schematic view illustrating additional configurations of air inlet openings for actuators according to further embodiments.

Fig. 27A and 27B respectively are diagrams representing delivery characteristics of actuators according to various embodiments which have two or three air inlet openings located in an actuator base.

Fig. 28 is a schematic view illustrating additional configurations of air inlet openings for actuators according to further embodiments.

Fig. 29 is a diagram representing delivery characteristics of actuators according to various embodiments which have two air inlet openings located in an actuator base, for different separation distances between centers of the air inlet openings.

Fig. 30 is a diagram representing delivery characteristics of actuators according to various embodiments which have one or two air inlet openings located in an actuator base, for different distances of a valve stem block orifice from the actuator base.

Fig. 31 is a diagram representing delivery characteristics of actuators according to various embodiments which have two or three air inlet openings located in an actuator base, in comparison with the delivery characteristics of actuators according to embodiments which have an additional air inlet opening in a rear wall of the actuator.

Fig. 32 is a diagram representing delivery characteristics for actuators according to embodiments, measured with an Andersen Cascade impactor (ACI).

Fig. 33 is a diagram representing delivery characteristics for the actuators according to the embodiments, measured with an Andersen Cascade impactor (ACI), for another formulation.

Fig. 34 is a diagram representing delivery characteristics of actuators according to embodiments, measured with an Andersen Cascade impactor (ACI), for yet another formulation.

Fig. 35 is a diagram representing a particle size distribution measured for actuators according to various embodiments, compared to the particle size distribution for a conventional actuator.

Fig. 36 is a diagram representing delivery characteristics of an actuator according to an embodiment for a suspension formulation containing ethanol, measured with an Andersen Cascade impactor (ACI).

Fig. 37 is a diagram representing a particle size distribution measured for an actuator according to an embodiment, compared to the particle size distribution measured for a control actuator, when delivering the suspension formulation containing ethanol.

Fig. 38 is a diagram representing a delivered dose as a function of a volumetric flow rate through the actuator for an actuator according to an embodiment.

Fig. 39 is a diagram representing actuator deposition as a function of a volumetric flow rate through the actuator for the actuator according to the embodiment.

Fig. 40 is a schematic cross-sectional view of a metered-dose inhaler including a conventional actuator.

DETAILED DESCRIPTION OF EMBODIMENTS

Exemplary embodiments of the invention will now be described with reference to the drawings. The features of the embodiments may be combined with each other unless specifically stated otherwise.

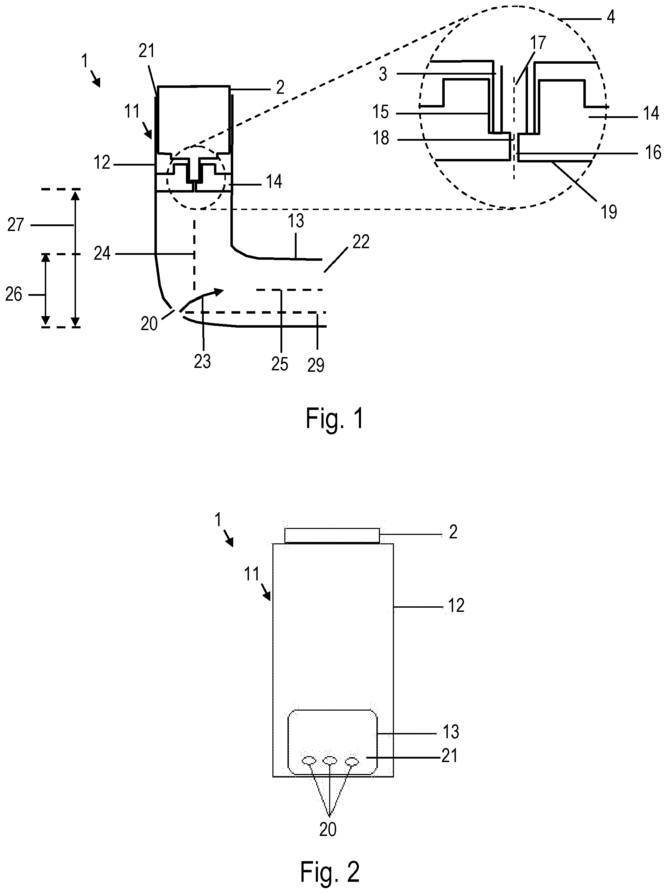

Fig. 1 is a schematic cross-sectional view of a metered-dose inhaler (MDI). The cross-sectional view is taken along the center symmetry plane of the MDI. The inset 4 in Fig. 1 illustrates a detail view of a valve stem block. Fig. 2 is a front view of the MDI as seen along a longitudinal axis of a mouthpiece portion

The MDI 1 includes a canister 2 and an actuator 11. The canister 2 contains an aerosol formulation. The aerosol formulation may be an aerosol solution formulation or an aerosol suspension formulation. The aerosol formulation may contain at least one active ingredient in a propellant or in a propellant/solvent system and, optionally, further excipients. The canister may be configured as a conventional canister for a pressurized MDI (pMDI). The canister 2 is provided with a valve having a valve stem 3. The valve may be a metering valve, which allows a metered dose to be dispensed through the hollow valve stem 3 upon actuation.

The actuator 11 has a housing which defines a canister receiving portion 12 and a mouthpiece portion 13. The canister receiving portion 12 is configured to receive the canister 2, which is at least partially inserted into the housing of the actuator 11 through an opening 21 for receiving the canister. The mouthpiece portion 13 defines a mouthpiece opening 22 through which an aerosol cloud may be dispensed.

The actuator 11 includes a valve stem block 14. The valve stem block 14 may be integrally formed with the housing of the actuator 11. The valve stem block 14 defines a valve stem receptacle 15 in which a front end of the valve stem 3 of the canister 2 is received. An orifice 16 is formed in the valve stem block 14. The orifice 16 extends to a face 19 of the valve stem block 14 which is opposite to the face on which the valve stem receptacle 15 is formed. The shape of the orifice 16 may be selected from a variety of shapes. For exemplary illustration, a cylindrical orifice 16 is shown in Fig. 1.

For the administration of a medicament through an MDI, a patient places the end of the mouthpiece portion 13 against his lips and actuates the MDI by depressing the canister 2 into the actuator 11. Alternatively, the MDI may be a breath actuated inhaler (BAI), which is configured to automatically actuate delivery of a dose of aerosol when the patient inhales with his lips in contact with the mouthpiece, without requiring additional manual actuation. Upon actuation, a metered dose, measured by the valve, is expelled from the valve stem 3. The expelled dose passes through an internal nozzle channel formed by the orifice 16 in the valve stem block 14. Upon passage through the orifice 16, the aerosol formulation is atomized. The patient starts the inhalation through the mouthpiece upon the release of the metered dose following the actuation of the MDI.

In the actuator 11, the valve stem block 14 is disposed so as to be spaced from an actuator base, which is defined by the lower boundary of the mouthpiece portion 13 when the MDI 1 is held in its use position, as illustrated in Figs. 1 and 2. The valve stem block 14 is disposed above the longitudinal axis 25 of the mouthpiece portion 13. In the illustrated embodiment, the valve stem block 14 is disposed at a distance 27 from the actuator base. The distance 27 is greater than a height 26 of the mouthpiece opening 22, measured from the actuator base. The valve stem block 14 is thus disposed so that it is not visible when the MDI is viewed from the mouthpiece opening 22, in a viewing direction parallel to a longitudinal axis 25 of the mouthpiece portion 13.

The distance 27 represents a base height 27, which is the distance between the face 19 of the valve stem block 14 and the actuator base. The base height may be defined as the distance between a plane in which an exit of the orifice 16 is located and the actuator base, measured along the rear wall of the actuator.

As best seen in the inset 4 in Fig. 1, the orifice 16 is formed in the valve stem block 14 such that a longitudinal axis 18 of the orifice 16 is aligned with a longitudinal axis 17 of the valve stem receptacle 15. The longitudinal axis 17 of the valve stem receptacle may coincide with a longitudinal axis 24 of the container receiving portion. As used herein, the term "longitudinal axis" refers to a center longitudinal axis of the respective concavity or component.

The valve stem block 14 is provided in the housing so as to extend throughout an inner cross section area of the actuator, with the exception of the orifice 16. The valve stem block 14 is configured to block passage of gas past the valve stem block 14 at any position located radially outwardly of the orifice 16. In particular, the valve stem block 14 does not include any air vents to allow the passage past the valve stem block 14, when the valve stem 3 is received in the valve stem receptacle. When the canister 2 is inserted into the canister receiving portion 12 and the valve stem 3 is received in the valve stem receptacle 15, air is substantially prohibited from passing from the container receiving opening 21 toward the mouthpiece opening 22.

One air inlet opening or a plurality of air inlet openings 20, or air vents 20, are formed in the outer shell of the actuator housing. The terms air vents and air inlet openings will be used synonymously. In use of the MDI, an inflow of air 23 will be established through the air inlet openings 20 by the inspiratory effort of the patient. The air inlet openings 20 are provided at a location which is spaced from both the container receiving opening 21 and the mouthpiece opening 22.

In the actuator 11, the air inlet openings 20 are provided on a part of the actuator housing which extends from the valve stem block 14 towards the mouthpiece opening 22. I.e., the air inlet openings 20 are provided downstream of the exit opening of the orifice 16, so that, in use of the MDI, respirable particles or droplets may be entrained in a flow 23 of moving air passing through the air inlet openings 20 into the actuator interior during an inhalation process.

Three air inlet openings 20 are shown in Fig. 2 for illustration. However, the number, shape and arrangement of the air inlet openings may be varied over a wide range. Embodiments of the invention are not limited to the particular number, shape and arrangement of air inlet openings 20 illustrated. Rather, a wide variety of numbers, geometries, sizes and positions of air inlet openings may be implemented in embodiments.

In the actuator 11, the air inlet openings 20 are provided on a rear wall of the actuator housing and in proximity to the actuator base. The term "rear wall" refers to the wall located opposite to the mouthpiece opening 22. The air inlet openings 20 are disposed such that each one of the air inlet openings 20 is in direct communication with the mouthpiece opening 22. A straight line 29, parallel to the longitudinal axis 25 of the mouthpiece and passing through one of the air inlet openings 20, intersects the mouthpiece opening 22 without passing through any solid portion or element of the actuator.

When the MDI 1 is used for dispensing aerosol formulation from the canister 2, the atomized spray is emitted from the orifice 16 along the longitudinal axis 18 of the orifice 16, which coincides with the longitudinal axis 17 of the valve stem receptacle and valve stem 3. Air is drawn into the actuator housing through the air inlet openings 20, by the inspiratory effort of the patient during inhalation. A flow 23 of moving air is generated, which passes across the actuator base. Respirable particles or droplets produced from the atomization of the formulation upon depressing the canister 2 into the actuator 11 are entrained in the airflow. Non-respirable particles or droplets are less likely to be entrained by the airflow, and are more likely to impact on the actuator base.

In the actuator 11, the air inlet openings 20 allow respirable particles or droplets produced from the atomized spray to be entrained, whilst non-respirable particles or droplets are more likely to impact on an inner actuator wall and to be retained within the actuator. The proportion of respirable particles or droplets relative to the non-respirable particles or droplets may be enhanced by this configuration.

Various modifications of the actuator 11 may be implemented in further embodiments. For illustration, other numbers, sizes, geometries or arrangements of the air inlet openings 20 may be implemented. For further illustration, the angle between the longitudinal axis 25 of the mouthpiece portion 13 and the longitudinal axis 24 of the canister receiving portion 12 may be included in the interval from 90° to 180°. The angle between the mouthpiece portion 13 and the longitudinal axis 24 of the canister receiving portion 12 may preferably be included in the range from 90° to 130°, and more preferably in the range from 90° to 110°.

Further, while a cylindrical orifice 16 is formed in the valve stem block 14, other shapes of orifices may be implemented in further embodiments. The arrangement of the orifice 16, with its longitudinal axis aligned with the valve stem longitudinal axis, allows orifice designs having a shape tapering towards the face 19 of the valve stem block 14 to be realized.

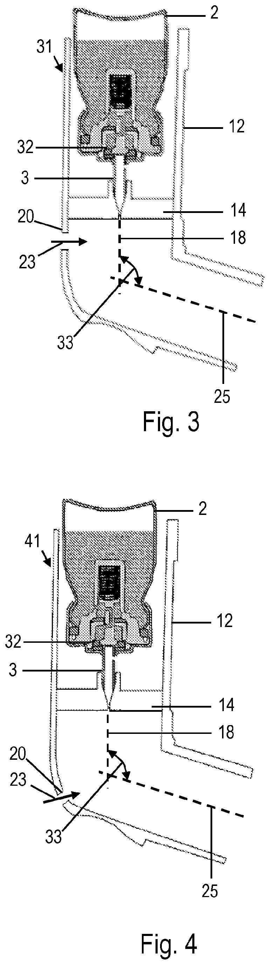

Fig. 3 is a schematic cross-sectional view of a metered-dose inhaler (MDI). The cross-sectional view is taken along the center symmetry plane of the MDI. Elements or features which correspond, with regard to their configuration and/or function, to elements or features of the MDI 1 of Figs. 1 and 2 are designated by the same reference numerals.

The MDI includes a canister 2 and an actuator 31. The canister 2 contains an aerosol formulation. The canister 2 has a valve assembly 32 which includes a valve stem 3.

The actuator 31 has a valve stem block 14 which defines a valve stem receptacle and an orifice. The valve stem block 14 extends across an inner cross section area of the actuator, so as to block passage of gas past the valve stem block 14 at all positions radially outwardly of the orifice. The longitudinal axes of the valve stem receptacle and orifice are aligned with each other. The orifice has a tapering portion. The tapering portion, which may be frustoconical, tapers in a direction away from the valve stem receptacle (i.e., in a downward direction in Fig. 3), i.e., in the downstream direction of the aerosol flow path. Producing an actuator with an orifice tapering in a downstream direction of aerosol flow is facilitated by the arrangement in which the longitudinal axis of the orifice is aligned with the longitudinal axis of the valve stem receptacle.

One or plural air inlet openings 20 are formed in the outer shell of the actuator housing. The air inlet openings 20 are spaced from the actuator base, and are disposed in proximity to the valve stem block 14. The air inlet openings 20 are formed in a rear wall of the actuator housing, which extends cylindrically around the longitudinal axis of the valve stem receptacle and the longitudinal axis 18 of the orifice.

The actuator 31 is configured such that an angle 33 between the longitudinal axis of the mouthpiece portion 12 and the longitudinal axis 18 of the orifice, which corresponds to the longitudinal axis of the canister 2 when the canister 2 is inserted into the actuator 31, is equal to or greater than 90°.

Fig. 4 is a schematic cross-sectional view of a metered-dose inhaler (MDI). The cross-sectional view is taken along the center symmetry plane of the MDI. Elements or features which correspond, with regard to their configuration and/or function, to elements or features of the MDI of Fig. 3 are designated by the same reference numerals.

The MDI includes an actuator 41 and a canister 2. A valve stem block 14 is provided in the actuator housing. An orifice formed in the valve stem block 14 tapers in a downstream direction of the aerosol flow. An angle 33 between the longitudinal axis of the mouthpiece portion 12 and the longitudinal axis 18 of the orifice, which corresponds to the longitudinal axis of the canister 2 when the canister 2 is inserted into the actuator 41, is greater than 90°.

In the actuator 41, one or plural air inlet openings 20 are formed in an outer shell of the actuator housing. The air inlet openings 20 are formed in proximity to the actuator base.

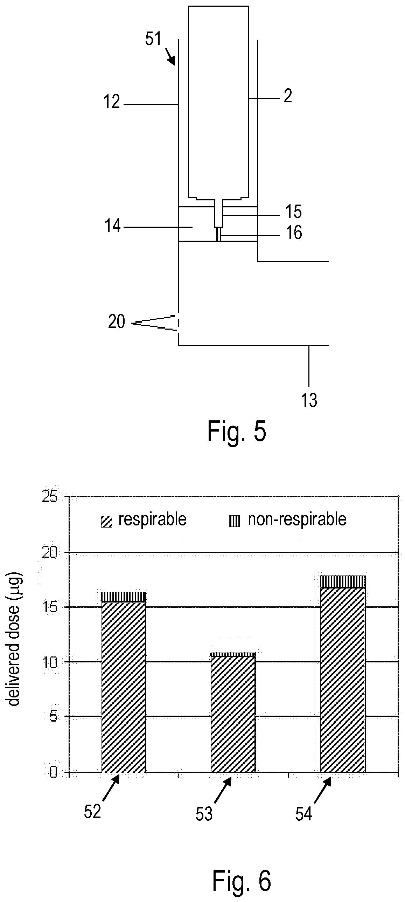

Fig. 5 is a schematic cross-sectional view of a metered-dose inhaler (MDI). The cross-sectional view is taken along the center symmetry plane of the MDI. Elements or features which correspond, with regard to their configuration and/or function, to elements or features of the MDI 1 of Figs. 1 and 2 are designated by the same reference numerals.

The MDI includes an actuator 51 and a canister 2. A valve stem block 14 is provided in the actuator housing. A cylindrical orifice 16 is formed in the valve stem block 14. A mouthpiece portion 13 of the actuator housing is disposed at an angle of approximately 90° relative to the canister receiving portion 12.

A plurality of air inlet openings 20 are formed in a rear wall of the actuator 51. At least two of the air inlet openings 20 are spaced along the longitudinal axis of the canister receiving portion 12. The air inlet openings 20 are formed in proximity to the actuator base, so as to be visible from the mouthpiece opening. In other words, the air inlet openings 20 are disposed to be in direct communication with the mouthpiece opening, there being no solid parts of the actuator interposed between the air inlet openings 20 and the mouthpiece opening.

Various other configurations of air inlet openings may be implemented in actuators according to further embodiments. For illustration, one or plural air inlet openings may be formed in the actuator base, in addition or alternatively to air inlet opening(s) being provided in the actuator rear wall. The one or plural air inlet opening(s) provided in the actuator base may be located so as to face the valve stem block.

In MDI actuators according to the embodiments explained above, an orifice formed in a valve stem block is arranged such that its longitudinal axis is aligned with the longitudinal axis of a valve stem receptacle. Air inlet openings are provided in the outer shell of the actuator housing, through which air is drawn into the actuator during inhalation. The resulting airflow may entrain a significant portion of respirable particles or droplets of the atomized formulation. A significant portion of non-respirable particles or droplets of the atomized formulation may impact on an interior surface of the actuator. The fraction of non-respirable particles or droplets in the aerosol cloud may be reduced before the aerosol cloud is dispensed via the mouthpiece opening.

Fig. 6 is a diagram illustrating the delivered dose. For distinction, Fig. 6 shows the respirable dose (fine particle dose), which is the amount of particles having an aerodynamic diameter of ≤5µm delivered on actuation of the inhaler, and the non-respirable dose, which is the amount of particles having an aerodynamic diameter of >5µm delivered on actuation of the inhaler containing a solution formulation of beclometasone dipropionate (BDP) (50µg/50µL) 8%w/w ethanol and up to 100 % w/w HFA 134a (1,1,1,2-tetrafluoroethane) propellant.

The delivered dose and respirable dose were respectively evaluated by an Andersen Cascade impactor fitted with a USP throat (Apparatus 1, United States Pharmacopoeia - USP34-NF29). Drug deposition in each stage was quantified by UPLC/MS (Ultra-Performance Liquid Chromatography/Mass Spectrometry).

At 52, the delivered respirable dose and non-respirable dose is shown for an actuator in which the exit opening of the orifice formed in the valve stem block is located at a distance of 22 mm above the base of the actuator, the distance being measured along the longitudinal axis of the container receiving portion. Three air inlet openings are provided in a rear wall of the actuator, as illustrated for the configuration of Figs. 1 and 2. The air inlet openings respectively have a circular cross section and a diameter of 3 mm, resulting in a total cross-sectional area of the air inlet opening of 21,2 mm$^2$.

The data indicated at 53 and 54 are obtained for actuators which do not include air inlet openings in the outer shell of the actuator housing at locations spaced from the canister receiving opening and the mouthpiece opening. The data indicated at 53 are obtained for an actuator in which the exit opening of the orifice formed in the valve stem block is located at a distance of 22 mm above the base of the actuator, the distance being measured along the longitudinal axis of the container receiving portion. The data indicated at 54 are obtained for an actuator in which the exit opening of the orifice formed in the valve stem block is located at a distance of 42 mm above the base of the actuator, the distance being measured along the longitudinal axis of the container receiving portion.

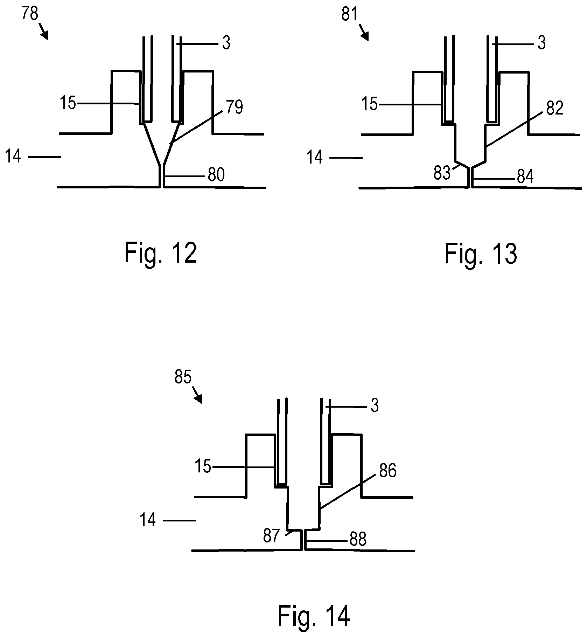

In each one of the actuators which have been used to acquire the data 52-54, the valve stem block is disposed spaced from an actuator base, and the longitudinal axis of the orifice formed in the valve stem block is aligned with a longitudinal axis for a valve stem receptacle. A cylindrical internal expansion chamber is formed in between the valve stem receptacle and the cylindrical orifice, as shown in Fig. 13. The orifice dimensions are identical for the three actuators for which the data 52-54 have been obtained.

As can be seen from data 52, 53 and 54 in Fig. 6, an actuator configuration in which the longitudinal axis of the orifice is aligned with the longitudinal axis of the valve stem receptacle has the effect that only a small fraction of non-respirable particles is entrained in the aerosol cloud output via the mouthpiece orifice. Non-respirable particles are more likely to impact the inner surface of the actuator housing when the longitudinal axis of the orifice is aligned with the longitudinal axis of the valve stem receptacle, as compared to designs in which the longitudinal axis of the orifice is aligned with the mouthpiece axis.

As can be seen from a comparison of data 52 and data 53 in Fig. 6, provision of the air inlet openings in the outer shell of the actuator housing allows the respirable dose (data indicated at 52 for an actuator having air inlet openings) to be increased as compared to the case in which there are no such air inlet openings in the outer shell of the actuator housing (data indicated at 53 for an actuator having no air inlet openings).

As can be seen from a comparison of data 52 and data 54 in Fig. 6, provision of the air inlet openings in the outer shell of the actuator allows the respirable dose (data indicated at 52) to be matched to the respirable dose obtainable for an actuator having a greater orifice-actuator base distance (data indicated at 54), but no air inlet openings. For a desired respirable dose, provision of the air inlet opening(s) in the outer shell of the actuator housing allows an actuator design to be realized in which the outer dimensions of the actuator are made to essentially correspond to the ones of a conventional actuator.

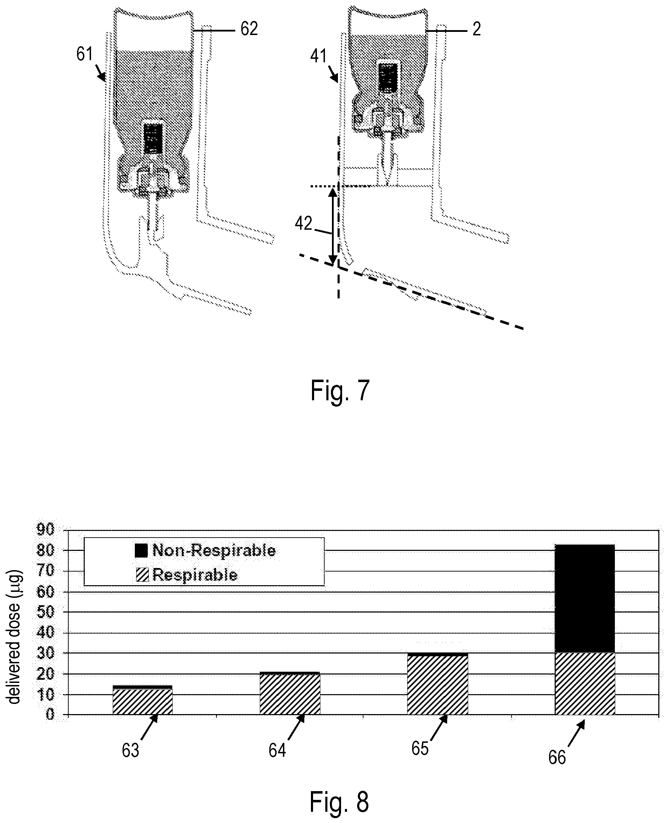

Fig. 7 exemplarily illustrates that the actuator according to various embodiments may be provided with outer dimensions corresponding to the outer dimensions of a conventional actuator 61 (shown on the left). For illustration, the actuator design 41 of Fig. 4 is shown in Fig. 7 (shown on the right), but actuator sizes comparable, or identical, to conventional actuator sizes may be attained for actuators according to any one of the embodiments described with reference to Figs. 1-5.

As has been explained with reference to Fig. 6, provision of one or plural air inlet opening(s) in the outer shell of the actuator at a position spaced from the canister receiving opening and the mouthpiece opening has the effect that a desired respirable dose may be obtained for a smaller distance 42 of the orifice formed in the valve stem block from the actuator base, as compared to an actuator having no air inlet openings formed in the outer shell thereof.

The distance 42 represents a base height distance 42. The base height distance is defined as the distance between the plane of a face of the member in which the exit of the orifice is located and the actuator base, measured along a rear wall of the actuator and parallel to the longitudinal axis of the orifice.

The actuator 41 of an embodiment may thus be configured to have external dimensions corresponding to a conventional actuator, indicated at 61 in Fig. 7.

The MDI according to an embodiment, with the canister 2 received in the container receiving portion of the actuator, may be configured such that it has external dimensions comparable, or identical, to a conventional MDI assembled from the actuator 61 and a canister 62. To attain this, a canister 2 having a reduced volume may be used. For illustration, a canister 2 having a capacity of 10-14 mL may be used in combination with an actuator according to an embodiment.

Fig. 8 is a diagram showing the respirable dose (fine particle dose), i.e. the amount of delivered particles having an aerodynamic diameter of ≤5μm, and non-respirable dose obtained from a solution formulation of beclometasone dipropionate (BDP) (100μg/50μL) 12%w/w ethanol and up to 100% w/w HFA 134a (1,1,1,2-tetrafluoroethane) propellant.

Data 63, 64 and 65 have been obtained using actuators in which a valve stem block is disposed at a distance from the actuator base and a longitudinal axis of the orifice formed in the valve stem block is aligned with the longitudinal axis of the valve stem receptacle. A cylindrical internal expansion chamber is formed in between the valve stem receptacle and the cylindrical orifice, as shown in Fig. 13. The orifice dimensions are identical for the three actuators for which the data 63, 64 and 65 have been obtained.

Data 63 has been obtained for an actuator which does not have air inlet openings in an outer shell of the actuator housing at positions spaced from the container receiving opening and the mouthpiece opening. The actuator has a mouthpiece disposed at an angle greater than 90°, and in particular of about 98°, relative to a longitudinal axis of the valve stem receptacle.

Data 64 has been obtained for an actuator which does not have air inlet openings in an outer shell of the actuator housing at positions spaced from the container receiving opening and the mouthpiece opening. The actuator has a mouthpiece wherein the angle was increased above 110° relative to the longitudinal axis of the valve stem receptacle.

Data 65 has been obtained for an actuator which has three circular air inlet openings formed in an outer shell of the actuator housing. Each one of the air inlet openings is circular having a diameter of 3 mm. The air inlet openings are provided in an actuator base. The actuator has a mouthpiece disposed at an angle greater than 90°, and in particular of about 98°, relative to the longitudinal axis of the valve stem receptacle. The data 65 have been obtained for an actuator with an outer shell which is generally similar to that of the actuator of Fig. 4, with the air inlet openings being positioned slightly further towards the mouthpiece opening.

Data 66 has been obtained for a conventional actuator as illustrated in Fig. 40. The conventional actuator has a valve stem block disposed on the actuator base. An orifice formed in the valve stem block has a longitudinal axis directed towards the mouthpiece opening. The orifice diameter of the conventional actuator has been identical to the orifice diameters of the actuators for which data 63, 64 and 65 have been obtained.

As can be seen from data 63-66, an actuator configuration in which the longitudinal axis of the orifice is aligned with the longitudinal axis of the valve stem receptacle (data 63, 64 and 65) has the effect that the fraction of non-respirable particles entrained in the aerosol cloud output via the mouthpiece orifice can be reduced as compared to the conventional design (data 66). Non-respirable particles are more likely to impact the inner surface of the actuator housing when the longitudinal axis of the orifice is aligned with the longitudinal axis of the valve stem receptacle, so that a large fraction of non-respirable particles may be removed from the aerosol cloud prior to the aerosol cloud exiting the mouthpiece opening.

As can be seen from a comparison of data 65 with data 63, the provision of air inlet openings in an actuator in which the longitudinal axis of the mouthpiece portion is disposed at an angle of greater than 90° relative to the longitudinal axis of the valve stem receptacle, or the longitudinal axis of the orifice, surprisingly increases the delivered dose of respirable particles.

As can be seen from a comparison of data 65 with data 66, the provision of air inlet openings in the actuator outer shell and the arrangement of the longitudinal axis of the mouthpiece portion at an angle of greater than 90° relative to the longitudinal axis of the valve stem receptacle significantly reduced the non-respirable dose compared to a convention actuator and contributed to the respirable dose being matched to that of a conventional actuator.

The actuators of the various embodiments allow concavities to be formed in the valve stem block 14 with a wide variety of shapes. The actuators of various embodiments allow a wide variety of orifice shapes to be defined without requiring that the valve stem block 14 is separately formed and later inserted into the housing of the actuator. While exemplary valve stem receptacle and orifice geometries are shown in Fig. 1-5, a great variety of different orifice, expansion chamber and valve stem receptacle designs may be implemented for any one of the actuator geometries described herein.

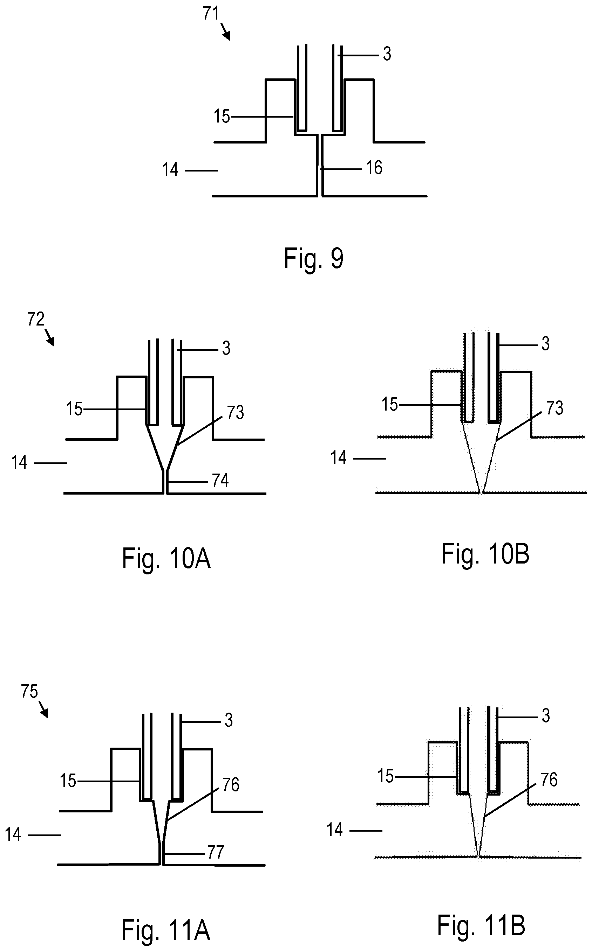

Figs. 9-14 show cross sectional views of center portions of a valve stem block 14 with the valve stem 3 received in the valve stem receptacle 15. The various geometries of concavities explained with reference to Figs. 9-13 may be implemented in the valve stem block of any one actuator described herein.

Fig. 9 shows a cross sectional view 71 of a valve stem block 14 of an actuator according to an embodiment. The valve stem block 14 defines a cylindrical valve stem receptacle 15. The valve stem block 14 further defines a cylindrical orifice 16 for atomizing formulation dispensed from the valve stem 3. The orifice 16 may be formed as a rotationally symmetrical orifice, i.e. with a cylindrical shape having a circular base.

Fig. 10A shows a cross sectional view 72 of a valve stem block 14 of an actuator according to an embodiment. The valve stem block 14 defines a cylindrical valve stem receptacle 15. An orifice having a tapering portion 73 and a cylindrical portion 74 is formed in the valve stem block 14. The tapering portion 73 may serve as abutment for the valve stem 3. The tapering portion 73 may have a frustoconical shape. The cylindrical portion 73 may be formed as a rotationally symmetrical portion, i.e. with a cylindrical shape having a circular base.

In the valve stem block 14 of Fig. 10A, the portion 73 tapers in a downstream direction of aerosol flow, i.e., towards the face of the valve stem block 14 opposite the valve stem receptacle 15. Such a tapering geometry can be readily realized in producing the actuator using conventional molding or other manufacturing techniques.

Fig. 10B shows a cross sectional view of a valve stem block 14 of an actuator according to an embodiment. The valve stem block 14 defines a cylindrical valve stem receptacle 15. An orifice is formed in the valve stem block 14 which has a tapering portion 73 corresponding to that of Fig. 10A, but without a terminal cylindrical portion at the interface with the mouthpiece.

Fig. 11A shows a cross sectional view 75 of a valve stem block 14 of an actuator according to an embodiment. The valve stem block 14 defines a cylindrical valve stem receptacle 15. An orifice having a tapering portion 76 and a cylindrical portion 77 is formed in the valve stem block 14. The tapering portion 77 may have a frustoconical shape. The cylindrical portion 77 may be formed as a rotationally symmetrical portion, i.e. with a cylindrical shape having a circular base.

In the valve stem block 14 of Fig. 11A, the maximum diameter of the tapering portion 76 is matched to an inner diameter of the valve stem 3. I.e., the tapered surface defining the tapering portion 76 is adjusted to the internal edge of the hollow valve stem 3. A step may be formed at a top edge of the tapering portion 76 to serve as an abutment for the valve stem 3. This configuration may prevent deposition of drug within the orifice formed in the valve stem block 14. This configuration may also reduce the formation of eddy currents when an aerosol formulation containing a high concentration of polar compounds such as water or ethanol is dispensed from the valve stem 3.

Fig. 11B shows a cross sectional view of a valve stem block 14 of an actuator according to an embodiment. The valve stem block 14 defines a cylindrical valve stem receptacle 15. An orifice is formed in the valve stem block 14 which has a tapering portion 76 corresponding to that of Fig. 11A but without a terminal cylindrical portion at the interface with the mouthpiece.

Fig. 12 shows a cross sectional view 78 of a valve stem block 14 of an actuator according to an embodiment. The valve stem block 14 defines a cylindrical valve stem receptacle 15. An orifice having a tapering portion 79 and a cylindrical portion 80 is formed in the valve stem block 14. The tapering portion 79 may have a frustoconical shape. The cylindrical portion 80 may be formed as a rotationally symmetrical portion, i.e. with a cylindrical shape having a circular base.

In the valve stem block 14 of Fig. 12, the maximum diameter of the tapering portion 79 is matched to an outer diameter of the valve stem 3. I.e., the tapered surface defining the tapering portion 79 is adjusted to the outer edge of the hollow valve stem 3.

Fig. 13 shows a cross sectional view 81 of a valve stem block 14 of an actuator according to an embodiment. The valve stem block 14 defines a cylindrical valve stem receptacle 15. An expansion chamber, or sump, 82 is formed in the valve stem block 14. The expansion chamber 82 may have a cylindrical shape. The expansion chamber 82 may have a volume which is smaller than typical volumes of internal expansion chambers formed in conventional actuators, in which the nozzle block is arranged on the actuator base. The expansion chamber 82 has a smoothly tapering portion 83. The tapering portion 83 may have a frustoconical shape. A cylindrical orifice 84 may be formed in the valve stem block. The cylindrical orifice 84 may be formed as a rotationally symmetrical orifice, i.e. with a cylindrical shape having a circular base.

Fig. 14 shows a cross sectional view 85 of a valve stem block 14 of an actuator according to an embodiment. The valve stem block 14 defines a cylindrical valve stem receptacle 15. An expansion chamber, or sump, 86 is formed in the valve stem block 14. The expansion chamber 86 may have a cylindrical shape. The expansion chamber 86 has a lower side 87 extending transverse to the side walls of the expansion chamber 86. A cylindrical orifice 88 may be formed in the valve stem block. The cylindrical orifice 88 may be formed as a rotationally symmetrical orifice, i.e. with a cylindrical shape having a circular base.

Various modifications may be implemented in the stem block configurations. For illustration, according to yet further embodiments, the orifice may have an elliptical cross section. I.e., the orifice may be not rotationally symmetrical.

Various configurations of the stem block configurations illustrated in Figs. 9-14 include portions tapering in a downstream direction of aerosol flow, i.e., towards the face of the valve stem block which is arranged opposite to the valve stem receptacle 15. Such tapering geometries can be readily realized in producing the actuator using conventional molding or other manufacturing techniques. For illustration, a pin tapering towards the actuator base may be used when molding the actuator, so as to define the tapering surface. The pin may be withdrawn from the molded actuator in a direction away from the actuator base.

Tapering orifice geometries as illustrated in Figs. 10-13 may be utilized to improve atomization, in particular for aerosol formulations containing a high concentration of polar compounds, which may be one or more polar co-solvents such as an alcohol (i.e. ethanol), water or a glycol. Such formulations may allow for a higher drug loading as compared to many conventional pMDI solutions. Improving the fraction of drug that can be delivered as respirable particles or droplets from formulations containing a high concentration of polar compounds is a need in the art. The use of tapering orifice geometries may also increase the velocity of the atomized aerosol, leading to a spray pattern with a smaller cone angle.

When an orifice tapering in the downstream direction of aerosol flow is defined in the valve stem block, more efficient atomization may be attained at least for some formulations. Droplets of a smaller size than those produced with non-tapering orifices may be produced using the tapering orifice.

Using a valve stem block having a tapering orifice, with the longitudinal axis of the orifice being aligned with the longitudinal axis of the valve stem receptacle, in an actuator housing having air inlet openings in its outer shell, as described with reference to Figs. 1-8, may assist in increasing the fraction of respirable particles or droplets at least for certain types of formulations, such as formulations having a higher concentration of polar low volatile compounds. The flow of air across the base of the actuator which is provided by the air inlet openings formed in the outer shell of the actuator may entrain a larger amount of atomized droplets. The proportion of non-respirable particles or droplets, which are not entrained in the flow of air, may be decreased due to the non-respirable particles or droplets being likely to impact on the actuator base. The proportion of smaller droplets may thereby be increased, while preventing larger droplets from impacting the throat of the patient.

As can be seen from Figs. 10-13, tapering orifice designs may be implemented in actuators of various embodiments. The cross-sectional area of the orifice, as a function of position along the longitudinal axis of the orifice, may be a decreasing, although not necessarily steadily decreasing, function. The ratio of the orifice diameter at the face of the valve stem block opposite the receptacle 15 to the maximum orifice diameter may be smaller than 1:10. The ratio of the orifice diameter at the face of the valve stem block opposite the receptacle 15 to the maximum orifice diameter may be greater than 1:30.

While air inlet openings may be positioned in a rear wall of the actuator, at least one or all of the air inlet openings may also be positioned at the actuator base. The actuator base may be defined by the boundary of the mouthpiece portion which is arranged opposite from the canister receiving portion. I.e., the lower side of the mouthpiece portion may define the actuator base.

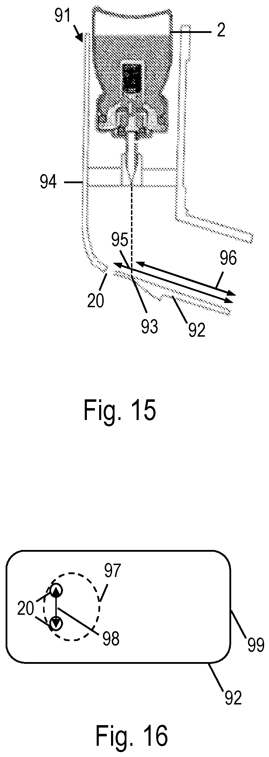

Fig. 15 is a schematic cross-sectional view of an MDI according to yet another embodiment. The MDI has an actuator 91 and a canister 2, which is insertable into a canister receiving portion of the actuator 91. The actuator 91 has a configuration generally similar to the one of the actuators of Figs. 1-5 and 7. A valve stem block 94 extends across a cross-section of the canister receiving portion. The valve stem block 94 may be configured to block passage of air radially outwardly of an orifice provided in the valve stem block 94. The valve stem block 94 and the orifice formed therein are arranged such that a longitudinal axis of the orifice is aligned with a longitudinal axis of a canister receiving portion of the actuator 91.

One or plural air inlet openings 20 are formed in the outer shell of the actuator 91. The air inlet opening(s) 20 are formed in an actuator base 92. The actuator base 92 is defined by the mouthpiece portion. When the actuator 91 is held in an operative position, in which the longitudinal axis of the canister receiving portion extends in a vertical direction and in which the canister is inserted, or can be inserted, into an upper end opening of the actuator, the actuator base 92 is defined by the lower side of the mouthpiece portion.

In the actuator 91, at least one air inlet opening 20 is arranged such that it is spaced from a rear wall 94 of the actuator 91.

The air inlet opening(s) 20 may be positioned in the actuator base 92 so that they are disposed towards the rear wall 94, relative to the virtual point of intersection 93 between the longitudinal axis of the orifice and the actuator base 92. The air inlet opening(s) 20 may be positioned in the actuator base 92 so that they are disposed towards the rear wall 94 relative to the impaction point of a plume which is dispensed upon actuation of the canister 2. In other words, a distance 95 from the air inlet opening to a mouthpiece opening, measured along a line parallel to a longitudinal axis of the mouthpiece, may be greater than a distance 96 from the point 93 to the mouthpiece opening, again measured along a line parallel to a longitudinal axis of the mouthpiece.

Such a configuration in which an air inlet opening or plural air inlet openings are positioned on the actuator base generates an air flow which, in proximity to the air inlet opening(s), is directed almost opposite to the direction of the plume. This may give rise to improved aerosol performance.

The positioning of the air inlet openings within the rear of the actuator, as illustrated in Fig. 1 or Fig. 2, produces an air flow essentially perpendicular to the direction of the plume. For air inlet openings positioned in the actuator base, the interaction between the plume and air flow may be increased in the sense that the air flow influences particle trajectories more strongly when the air inlet openings are provided in the actuator base. This may lead to reduced actuator deposition.

The positions of the air inlet opening(s) on the actuator base may be set further as a function of the lateral dimensions of an impaction area of the plume onto the actuator base. This is illustrated in Fig. 16.

Fig. 16 is a schematic plan view of an actuator base 92. At one longitudinal end, the actuator base 92 defines an edge of a mouthpiece opening 99. Two air inlet openings 20 are positioned on the actuator base 92. The air inlet openings 20 are offset from each other in a direction transverse to the longitudinal direction of the mouthpiece portion. A distance 98 between centres of the air inlet openings 20 may be set based on a size of an impaction area 97 in which the plume impacts onto the actuator base 92. The distance 98 may be set so that the air inlet openings 20 are arranged towards the edge of the impaction area 97. The distance 98 may be set based on a base height.

Additional air inlet opening(s) may be provided. For illustration, one additional air inlet opening may be positioned in the actuator base such that the three air inlet openings form a triangular arrangement or a linear arrangement.

The positions of the air inlet opening(s) on the actuator base may respectively be set as a function of base distance.

Various effects may be attained using MDI actuators, MDIs and methods of embodiments. For illustration, upon actuation of the canister the plume may be emitted along the common axis 16, 24 depicted in Figure 1. A significant fraction, or essentially all, of the non-respirable dose may be removed from the aerosol through internal impaction within the actuator, resulting in a high fine particle fraction (of particles with sizes $\leq 5\mu m$) which may be 90% or more. This may reduce oro-pharyngeal drug deposition and associated gastrointestinal side effects.

For further illustration, while the fraction of non-respirable particles may be reduced compared to a conventional actuator design, aerosol performance in the MDI having an actuator according to an embodiment is comparable to that of a conventional actuator for each formulation regardless of non-volatile content (%w/w). This applies to suspension formulations as well as solution formulations. This suggests that a selected design could be used successfully for different formulations.

While embodiments of MDI actuators have been described in detail with reference to the drawings, various modifications may be implemented in other embodiments. For illustration, while the arrangement of the orifice with its longitudinal axis being aligned with a longitudinal axis of the valve stem receptacle allows tapering orifice geometries to be realized, the orifice does not need to be provided with a tapering shape. The geometry of the orifice may be selected in accordance with the formulation to be dispensed.

For further illustration, the actuator of any one of the various embodiments may be configured as an actuator for a breath actuated inhaler (BAI). The actuator may include additional components to automatically trigger release of a dose of aerosol when the patient inhales with his/her lips in contact with the mouthpiece. The MDI according to various embodiments may be BAI.

While MDI actuators of embodiments having exemplary numbers, shapes, sizes and arrangements of air inlet openings have been explained in the context of illustrative embodiments, other numbers, shapes, sizes and arrangements of air inlet openings may be implemented in actuators according to further embodiments.

The MDI actuators and MDIs may be utilized for various aerosol formulations. For illustration, while actuators of some embodiments may be utilized for dispensing formulations containing a high concentration of polar low volatile compounds such as water, ethanol or a glycol, the actuators are not limited to this particular field of application.

While embodiments have been described in which a tapering orifice is formed in a valve stem block of an actuator which has air inlet openings in its outer shell at positions spaced from the canister receiving opening and the mouthpiece opening, the tapering orifice may also be implemented in other actuators. For illustration, an orifice tapering in a downstream direction of aerosol flow may be formed in a valve stem block integrated in the housing of an actuator, which does not have air inlet openings in its outer shell at positions spaced from the canister receiving opening and the mouthpiece opening.

For further illustration, MDIs according to various embodiments will be described in more detail with reference to examples.

EXAMPLES

SCREENING OF PRESSURIZED MDIs ACCORDING TO EMBODIMENTS

For the rapid screening of different actuators according to embodiments which have an in-line configuration (orifice axis aligned with a longitudinal axis of a canister receiving portion), determination of the delivered dose, fine particle fraction (%) and respirable dose (particles $\leq 5\mu m$) were performed with a Fast Screening Andersen (FSA) impactor (from Copley) at a flow rate of 28.3 ($\pm 5$%) L min-1.

The FSA is equipped with two stages $\leq 5\mu m$ and $\leq 1 \mu m$, and the filter. After a single shot was actuated into the assembled FSA, the mouthpiece and USP throat were rinsed to determine beclomethasone dipropionate (BDP) deposition. The collection plates and filter were removed from the FSA to determine BDP deposition at each stage. The FSA was then re-assembled with clean collection plates, throat and mouthpiece. A second shot was fired into the FSA and the sample collection repeated. After three actuations had been collected, the actuator and canister were disassembled and average actuator deposition determined for four shots. Samples were collected in 15:85 water:methanol solution and analysed by UPLC.

The FSA was used as a screening tool to rapidly assess in-line actuators for improvements in delivered dose, fine particle fraction, and fine particle dose ($\leq 5\mu m$) relative to the control. Controls were performed with a conventional actuator having an orifice diameter of 0.22 mm, using the FSA method described above, or with a conventional actuator having an orifice diameter of 0.30 mm.

Lead prototypes of the actuators of embodiments were further assessed using the Andersen Cascade Impactor (ACI) USP Apparatus 1 with induction port; USP34-NF29 at a flow rate of 28.3 ($\pm$5%) L min$^{-1}$ to identify differences in particle size distribution compared to the control.

Aerosol characteristics determined include mass median aerodynamic diameter (MMAD), i.e., the diameter around which the mass aerodynamic diameters of the emitted particles are distributed equally; the fine particle dose (FPD), corresponding to particles of diameter $\leq 5$ $\mu$m; the fine particle fraction (FPF) which is the percent ratio between the respirable dose and the delivered dose; and the extrafine particle dose and extrafine particle fraction, respectively, which correspond to particles of diameter $\leq 1$ $\mu$m collected in the ACI.

ACTUATOR PROTOTYPE DESIGN

The prototypes for actuators of embodiments used in the tests include a stem block having an orifice, with the longitudinal axis of the orifice being aligned with a longitudinal axis of the canister receiving portion of the actuator (also referred to as "in-line actuator", "in-line configuration" or similar below). The stem block was formed from aluminium. Lower and upper actuator portions of a conventional pMDI are fitted onto the valve stem block.

The orifice design of the stem block used in the experiments mainly corresponds to that of Fig. 13. The diameter of the orifice 84 was measured using stereo microscopy, giving an accurate diameter of 0.26 mm, for a length of about 0.6 mm. The expansion chamber 82 has a length of 7.02 mm and a diameter of 2.10 mm.

In the prototypes for actuators of embodiments, the angle between the longitudinal axis of the mouthpiece portion and the longitudinal axis of the canister receiving portion is 107°. Control actuators, i.e. conventional or standard actuators used for comparison, had the same angle between the two longitudinal axes.

FORMULATIONS

The different device designs were tested with the following beclomethasone dipropionate (BDP) formulations. These formulations provide different atomisation characteristics in terms of particle size distribution and evaporation rate. Each formulation was packaged in a standard aluminium 19 ml canister fitted with a conventional 50$\mu$L valve.

Table 1: Formulation compositions using HFA 134a (13.6g fill weight)

| Formulation | BDP Dose ($\mu$g/$\mu$L) | Ethanol content (%w/w) | Glycerol content (%w/w) | HFA 134a content (% w/w) |
|---|---|---|---|---|
| EF | 100/50 | 13 | - | 86.8 |
| LVC | 100/50 | 13 | 1.3 | 85.5 |
| HE | 100/50 | 26 | - | 73.8 |
| Low NVC | 6/50 | 13 | - | 86.99 |
| High NVC | 250/50 | 13 | - | 86,5 |

EF = Extrafine formulation (a formulation which is free from low volatility component);

LVC = Low Volatility Component formulation (a formulation which comprises glycerol as the low volatility component);

HE = High Ethanol content formulation (a formulation which has double ethanol concentration with respect that of EF or LVC concentration);

Low or high NVC = Formulations with low or high non-volatile content (i.e. formulations having a lower or higher concentration in active ingredient).

CONFIGURATIONS OF AIR INLET OPENINGS

Prototypes of actuators according to embodiments were designed which had different numbers, positions and sizes of air inlet openings (i.e., different vent designs), and which had different base heights. The effect of base height, vent design and total cross-sectional area of the air inlet openings on the performance of the actuator was determined with each of the test formulations.

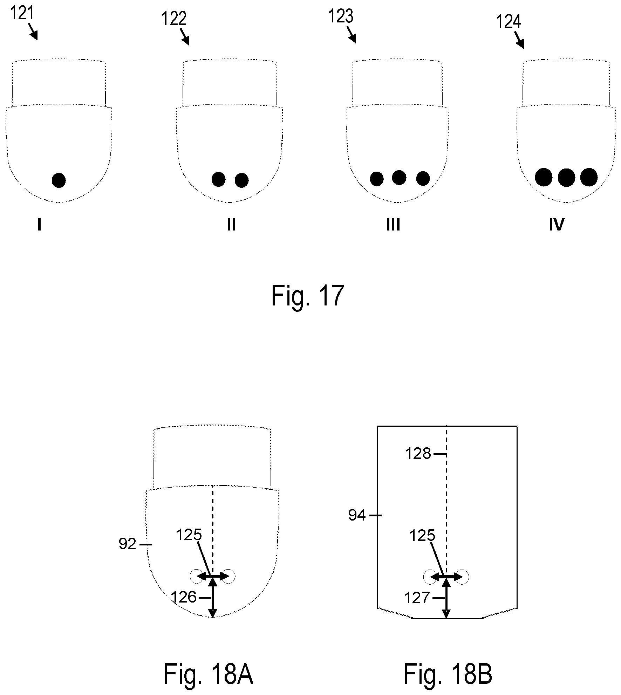

The main designs of air inlet openings (vent designs I, II, III, IV) utilized are shown in Figure 17.

Design I, shown at 121, has a single air inlet opening located either in a base or in a rear wall of the actuator. The diameter of the air inlet opening is 3.0 mm. The area of the air inlet opening is 7 mm$^2$.

Design II, shown at 122, has two air inlet openings located either in a base or in a rear wall of the actuator. The diameter of each air inlet opening is 3.0 mm. The total area of the air inlet openings is 14 mm$^2$.

Design III, shown at 123, has three air inlet openings located either in a base or in a rear wall of the actuator.

The air inlet openings have a linear arrangement. The diameter of each air inlet opening is 3.0 mm. The total area of the air inlet openings is 21 mm$^2$.

Design IV, shown at 124, has a three air inlet openings located either in a base or in a rear wall of the actuator. The air inlet openings have a linear arrangement. The diameter of each air inlet opening is 4.25 mm. The total area of the air inlet openings is 43 mm$^2$.

Additional prototypes for actuators according to yet other embodiments were manufactured for the study. The configurations of such actuators are described within the relevant sections within the results and discussion.

ASSESSING DIFFERENT CONFIGURATIONS OF AIR INLET OPENINGS

Rapid screening of actuators according to various embodiments was performed to assess the performance of different configurations of air inlet openings, for different distances of the orifice from the actuator base.

(a) Base heights

The base height is defined as the distance from the base of the actuator to the valve stem block (see distance 27 in Fig. 1 and distance 42 in Fig. 7, respectively measured as distance along the rear outside boundary of the housing from the housing base to the lower side of the member in which the orifice is formed; i.e. the base height may be defined as distance of the lower end of the rear wall of the actuator from the plane in which the exit opening of the orifice is located). Actuators having various distances between the orifice and the actuator base were manufactured, namely: 12 mm; 22 mm; 32 mm; 42 mm; 52mm.

Three base heights: 12 mm, 32 mm, and 52mm, representing the upper and lower extreme and a mid-point, were selected to identify which one of the various configurations of air inlet openings shows best performance (also referred to as "optimised design" herein, it being understood that the optimisation refers to the various different configurations of air inlets tested and need not represent a global optimum). For each base height, actuators having this base height were assessed for each of the vent designs and positions. Additional work was performed using a base height of 22 mm.

(b) Air inlet opening configurations and total cross-sectional area

Air inlet openings located in the lower portion of the actuator have been shown to improve the aerosol performance of the MDI using an actuator according to an embodiment. To establish the effect of arrangements of air inlet openings (vent designs) and total cross-sectional area on the aerosol performance of the formulations, the four different designs I-IV (see Fig. 17) corresponding to a total cross-sectional area of 7; 14; 21; or 43mm$^2$ were primarily utilised. The vent designs are illustrated in Figure 17. Prototypes for actuators having the various vent designs were manufactured for the three base heights of 12 mm, 32 mm, and 52mm.

(c) Position of air inlet openings

Two positions were investigated for the vent designs. The air inlet openings were located on the lower portion of the actuator, either in the actuator base or in the actuator rear.

For air inlet opening designs in which multiple air inlet openings are provided, a fixed distance of 5 mm between the centre points of air inlet openings was generally used.

Fig. 18A shows air inlet openings located in an actuator base 92. The air inlet openings were generally positioned at a distance 126 of 10 mm from the rear wall. The distance 125 between the centers of the air inlet openings was 5 mm. The air inlet openings were positioned to be parallel to the mouthpiece opening. The position of the air inlet openings was not altered unless otherwise stated.

Fig. 18B shows air inlet openings located in an actuator rear wall 94. The rear wall 94 is the wall extending generally parallel to the longitudinal axis of the canister receiving portion, at the side facing away from the mouthpiece portion. The air inlet openings were generally positioned a distance 127 of 10 mm from the actuator base. However, for actuators having a base height of 12 mm, with design I (one air inlet opening) and air inlet opening provided in the rear wall, the distance 127 was only 5mm. The distance 125 between the centers of the air inlet openings was 5 mm. The air inlet openings were positioned to be parallel to the top of the actuator, i.e., the canister receiving opening. The position of the air inlet openings was not altered unless otherwise stated. Measurements were performed for actuators having different base heights 128, i.e., different distances between the exit opening of the orifice and the actuator base.

(d) Device resistance

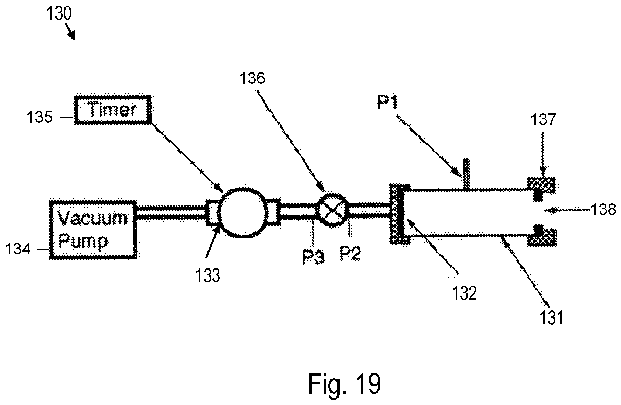

The device resistance, or pressure drop, is directly related to the pressure differential across the device that occurs when a flow rate is drawn through the in-line actuator. The device resistance also relates to the velocity of air flow at the air inlet openings. The pressure drop across the in-line prototype was measured using a sample collection tube with pressure tap (Apparatus B; Delivered Dose Uniformity- USP34-NF29) as shown in Fig. 19.

The apparatus 130 shown in Fig. 19 comprises: a sample collection tube 131, a filter 132, a two-way solenoid valve 133, a vacuum pump 134, a timer 135, a flow control valve 136, a mouthpiece adapter 137, and an inlet 138. P1, P2 and P3 represent pressure measurement points.

The actuator of an embodiment was seated in the inlet 138 of the apparatus 130 using a moulded mouthpiece. Air was drawn through the sample collection tube 131 using the vacuum pump 134 and the flow rate was adjusted to 28.3 ($\pm$5%) L min$^{-1}$ with the two-way solenoid valve 133. A differential pressure manometer was attached to the pressure tap P1 and the pressure drop across the device was measured in kPa using a differential manometer (Digitron).

PERFORMANCE OF DIFFERENT CONFIGURATIONS OF AIR INLET OPENINGS FOR VARIOUS BASE HEIGHTS

Each of the four vent designs I-IV (see Fig. 17) at three base heights of 12 mm, 32 mm and 52 mm was tested with the BDP (100/50) formulations (Table 1), i.e., with the EF, LVC, and HE formulations. Air inlet openings were either located on the base or on the rear wall of the lower actuator portion. Studies were conducted to determine the relationship between actuator design and performance.

(a) Air inlet openings located in actuator base

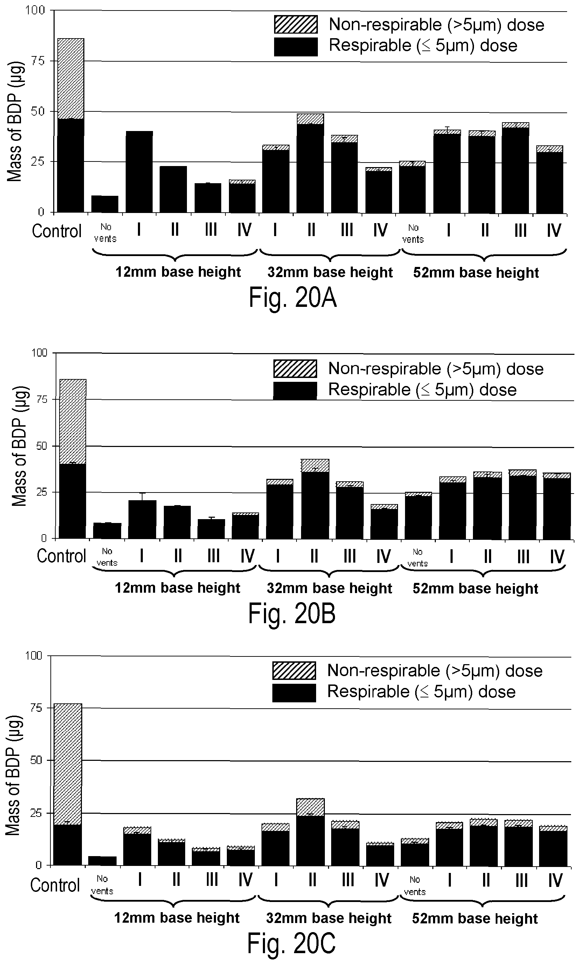

Fig. 20A-20C shows the aerosol performance of the BDP (100$\mu$g/50$\mu$L) extrafine, low volatility component, and high ethanol content formulations using the actuator of an embodiment with air inlet opening(s) located in the actuator base. The aerosol performance of the BDP (100/50) formulations (EF; LVC; HE) using the different actuator designs is given in Fig. 20A (for the extrafine formulation EF), Fig. 20B (for the low volatility component formulation LVC) and Fig. 20C (for the high ethanol content formulation, HE). For the data shown in Fig. 20A-20C, the actuator had air inlet openings located on the actuator base. The different vent designs I-IV shown in Fig. 17 were respectively used on actuators having base heights of 12 mm, 32 mm and 52 mm.

The use of the actuator of an embodiment reduces the non-respirable dose (>5$\mu$m) compared to the control for all designs and formulations. The control is a conventional actuator having an orifice diameter of 0.22 mm, for which the longitudinal axis of the orifice is not aligned with the longitudinal axis of the canister receiving portion.

At 12 mm base height, the absence of air inlet openings drastically reduces the delivered and respirable dose compared to the control. Increasing the base height to 52 mm improves the dose characteristics but fails to match the respirable dose (≤5$\mu$m) obtained from the control when no air inlet openings are present.

When air inlet openings are added to the design, with the inlet openings located in the actuator base, an improvement in the respirable dose is observed at each base height. The effect of vent design and total cross-sectional area is most notable at the lower base heights. For example, the introduction of a single air inlet opening (design I) at 12mm base height causes approximately a five-fold increase in respirable dose achieved with BDP (100/50) extrafine when compared to the use of no air inlet openings. The magnitude of this effect is reduced for the other formulations, the relative increase being greater for the extrafine (EF) formulation than for the high ethanol formulation (HE), and the effect being more pronounced for the high ethanol formulation (HE) than for the low volatility component formulation (LVC). This may likely be attributed due to the differences in droplet size at 12 mm base height that occur as a result of the inclusion of glycerol or the increase in ethanol concentration.

The use of vent design I results in a respirable dose equal to 81.8% and 77.5% of the conventional actuator for the extrafine (EF) and high ethanol (HE) formulations respectively. However, only 46.6% of the respirable dose of the conventional actuator is achieved when dispensing the LVC formulation. At the 12mm base height, an increase in total cross-sectional area of the air inlet opening(s) causes a corresponding decrease in respirable dose. Although this trend is observed for all formulations, the effect is attenuated for the LVC and high ethanol formulations.

When the base height is increased to 32 mm, the respirable dose increases between designs I and II, and subsequently reduces in line with the increase in total cross-sectional area of the air inlet opening(s) (design III and design IV). This trend is the same across all three formulations. The effect that determines the reduction in performance associated with increasing cross-sectional area at 12 mm base height is altered by the increase in base height to 32 mm. At this height, performance increases between vent design I and vent design II. The design with two air inlet openings (design II) in the base achieves the maximum respirable dose among the different vent designs evaluated.

The effect of the configuration of the air inlet openings and/or cross sectional area is reduced when the base height is extended to 52mm. Little or no difference is present between vent designs I, II and III. However, a slight reduction in performance is observed for the extrafine (EF) formulation when using design IV. This may be attributed to the increase in diameter of the air inlet opening from 3.0 mm to 4.25 mm and the subsequent effect this may have on air flow within the prototype.

The prototype design that performed the best for each formulation was the configuration having two air inlet openings in the base (design II in Fig. 17) at 32 mm base height. The aerosol characteristics of each formulation in comparison with a conventional MDI (orifice diameter 0.22 mm) as control are given in Table 2. The respirable dose achieved when using this in-line prototype is 95.3 %, 89.7 % and 122.1 % of that observed when using a conventional MDI for the extrafine (EF), low volatility content (LVC) and high ethanol (HE) formulations respectively.

Table 2: Aerosol characteristics of the BDP (100/50) test formulations when using the actuator with 32mm base height with vent design II (see Fig. 17) according to an embodiment ("In-line") compared with a conventional MDI (number of measurements: n=3; average $\pm$ standard deviation)

| Dose characteristics ($\mu$g) | EF | | LVC | | HE | |
|---|---|---|---|---|---|---|
| | In-line | control MDI | In-line | control MDI | In-line | control MDI |
| Metered dose | 104.1 (0.1) | 95.9 (2.7) | 99.6 (2.3) | 97.1 (1.5) | 95.6 (1.5) | 89.3 (1.0) |
| Delivered dose | 49.0 (0.2) | 85.9 (3.2) | 43.0 (2.3) | 86.0 (1.8) | 32.1 (1.5) | 76.9 (0.78) |
| Non-respirable dose (>5$\mu$m) | 5.1 (0.4) | 39.9 (3.1) | 6.9 (1.0) | 45.7 (1.8) | 8.2 (0.6) | 57.3 (0.9) |
| Respirable dose ($\leq$5$\mu$m) | 43.9 (0.5) | 46.0 (0.5) | 36.1 (1.9) | 40.3 (0.9) | 23.9 (0.9) | 19.6 (1.6) |
| Extrafine dose ($\leq$1$\mu$m) | 21.2 (0.4) | 21.3 (1.4) | 3.9 (0.0) | 4.3 (0.2) | 10.6 (0.7) | 8.1 (1.3) |

Using an actuator of an embodiment, the fraction of non-respirable particles can be reduced. The respirable dose may be essentially matched to that of a conventional actuator when using air inlet openings.

(b) Air inlet openings located in actuator rear wall

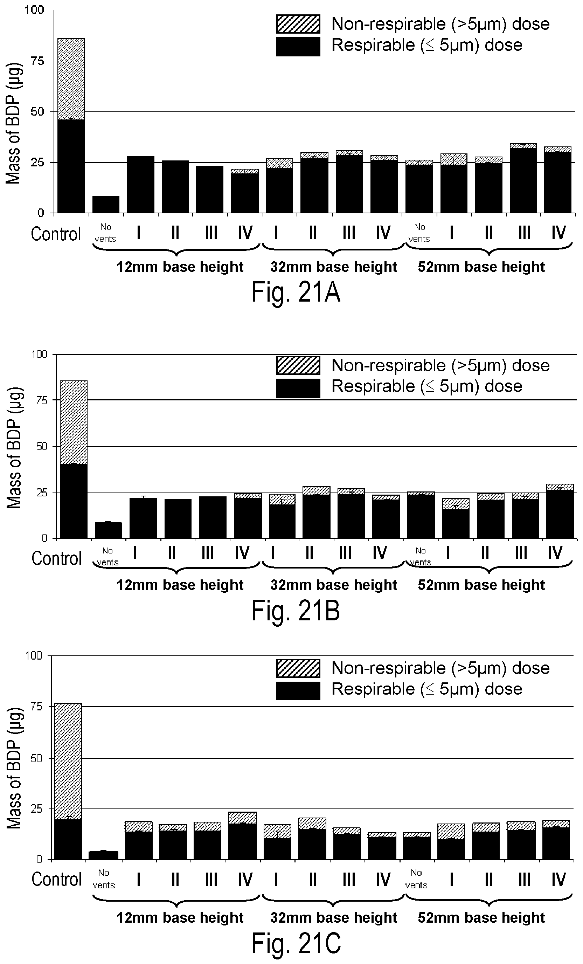

Fig. 21A-21C shows the aerosol performance of the BDP (100$\mu$g/50$\mu$L) extrafine (EF), low volatility component (LVC), and high ethanol (HE) content formulations using the actuator of an embodiment with air inlet opening(s) located in the actuator rear wall. The aerosol performance of the BDP (100/50) formulations (EF; LVC; HE) using the different actuator designs is given in Fig. 21A (for the extrafine formulation EF), Fig. 21B (for the low volatility component formulation LVC) and Fig. 21C (for the high ethanol content formulation HE). For the data shown in Fig. 21A-21C, the actuator had air inlet openings located on the actuator rear wall. The different vent designs I-IV shown in Fig. 17 were respectively realized for actuators having base heights of 12 mm, 32 mm and 52 mm.

As can bee seen from Fig. 21A-21C, the effect on the respirable dose caused by the introduction of air inlet openings in the rear is different to that observed when using air inlet openings in the base. For example, changing the vent design and total cross-sectional area has little effect on the respirable dose, even at the low base heights.

At the 12mm base height, a slight downward trend in respirable dose is observed with the EF (extrafine) formulation with increasing total cross-sectional area. The overall difference in the average respirable dose achieved between vent design I and vent design IV is 5.8$\mu$g. For comparison, the difference between vent design I and vent design IV for air inlet openings located in the base was 23$\mu$g.

For all other formulations, the respirable dose achieved between the designs is approximately within one standard deviation. Although little difference is observed between the designs, the introduction of air inlet openings in the actuator rear improves the performance compared to the prototype in which air inlet openings are absent.

While vent design has little impact on performance, the high ethanol (HE) formulation does achieve a respirable dose approaching that of the conventional MDI actuator. For the extrafine (EF) and low volatility component (LVC) formulations, the respirable dose is less than half that of the corresponding conventional actuator (control), but a significant reduction of the non-respirable dose is still observed. This observed difference in the behaviour of the formulations may be due to a reduction in the HFA content, which is 73.8%w/w (for the LVC formulation) compared with 86.8%w/w and 85.5%w/w found in the EV and LVC formulations, respectively.

When the base height is increased to 32 mm, there is no significant increase in the respirable dose achieved by the actuator. In some instances, most notably the high ethanol formulation, the dose has decreased. Likewise at 52 mm base height, an overall increase is not observed. At 52 mm the performance of the actuator is similar whether air inlet openings are included or absent.

(c) Summary of results

Generally, air inlet openings located in the actuator base produce a greater effect on the respirable dose achieved from the actuator according to some embodiments than air inlet openings located in a rear wall of the actuator.
The effect on the respirable dose obtained when using air inlet openings located in the actuator base changes as a function of base height. This effect may be related to the design of the pattern or the total cross-sectional area of the air inlet openings.
The influence of the vent design, which depends on the base height, is related to the evolution of the plume as a function of the base heights tested. The effect is not greatly influenced by the type of formulation.
Air inlet openings located in a rear wall produce a respirable dose that is less strongly affected by the vent design, total cross-sectional area or base height. The performance of the actuator having an in-line configuration with rear air inlet openings when compared to the conventional actuator is dependent on the formulation. For the high ethanol (HE) content formulation, a respirable dose matching that of the conventional MDI is attained.

FURTHER EXAMPLES ILLUSTRATING THE RELATIONSHIP BETWEEN VENT DESIGN AND PERFORMANCE

(a) Device resistance and air velocity at air inlet openings

To illustrate the effect of the vent design on the resistance to air flow associated with the actuator having an in-line configuration, pressure drop (kPa) was measured across the MDI.

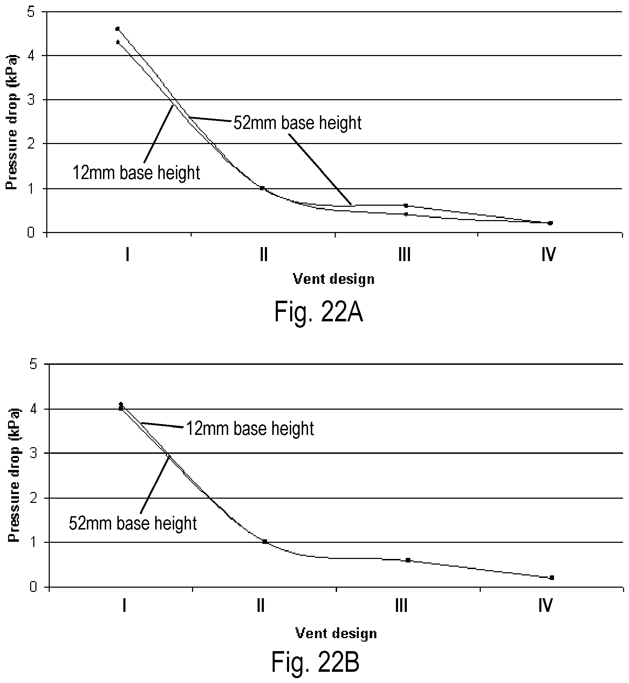

Fig. 22A shows the change in pressure drop across the MDI for the different vent designs I-IV, with the air inlet openings located in the actuator base. Fig. 22B shows the change in pressure drop across the MDI for the different vent designs I-IV, with the air inlet openings located in the actuator rear.
Neither base height (12 mm vs. 5 2mm) nor air inlet opening position (base vs. rear) had a significant effect on device resistance. High resistance was observed using the single 3.0 mm air inlet opening (vent design I in Fig. 17) but this was drastically reduced when a second 3.0 mm air inlet opening was introduced (vent design II in Fig. 17). The addition of a third air inlet opening (vent design III in Fig. 17) and an increase in opening diameter (vent design IV in Fig. 17) caused an additional, smaller reduction in device resistance.
The mean air velocity of each vent design was also calculated:

$$v = \frac{\left( \dfrac{Q \times 1000}{A \times n} \right)}{60},$$

where v is the mean air velocity (m s$^{-1}$); Q is the volumetric flow rate (L min$^{-1}$); A is the cross-sectional area of the air inlet opening (mm$^2$) and n is the number of air inlet openings. The calculated values are given in Table 3. The mean air velocity is inversely proportional to the total cross sectional area. Therefore, the expected mean air velocity reduces as the number of 3.0 mm diameter air inlet openings increases (vent design I-III illustrated in Fig. 17). An additional reduction occurs when the diameter of the air inlet openings is increased from 3.0 mm to 4.25 mm (from vent design III to vent design IV illustrated in Fig. 17).

Table 3: Mean air velocity at the air inlet opening calculated at a volumetric flow rate of 28.3 Lmin$^{-1}$.

| Vent design | Number of air inlet openings | Device pressure drop (kPa) | Total cross-sectional area (mm$^2$) | Calculated mean air velocity (m s$^{-1}$) |
|---|---|---|---|---|
| I | 1 | 4.3 | 7 | 66.7 |
| II | 2 | 1.0 | 14 | 33.4 |
| III | 3 | 0.6 | 21 | 22.2 |
| IV | 3 | 0.2 | 43 | 11.1 |

(b) Actuator having 12 mm base height and vent design I (see Fig. 17) with air inlet opening located in actuator base

To determine whether the pressure drop across the device was related to the reduction in respirable dose observed when using the vent designs located in the base at 12 mm base height, a range of actuators respectively having a base height of 12 mm were prepared. A single base vent was formed in the actuator base. The diameter of the base air inlet opening ranged between 3.0 mm and 4.5 mm at 0.5 mm intervals.

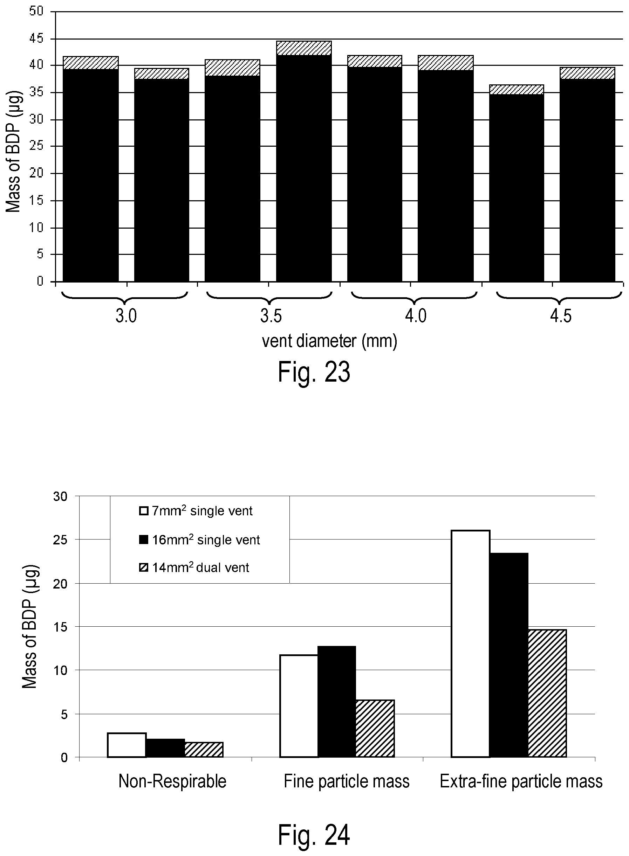

The pressure drop associated with these devices having a single air inlet opening ranged between ~4kPa and ~1kPa. The effect of decreasing pressure drop on the respirable dose obtained with BDP (100/50) extrafine (EF) formulation when dispensed using an actuator having a base height of 12mm with a single air inlet opening in the actuator base is given in Fig. 23.

Fig. 23 shows the aerosol performance of the BDP (100/50) extrafine (EF) formulation as a function of air inlet opening diameter, at a given base height of 12 mm. Two measurements were performed for each air inlet opening diameter.

Pressure drop decreases with increasing diameter of the base air inlet opening. However, there is no overall effect on the respirable dose.

The total cross-sectional area of a single air inlet opening in the base, with the air inlet opening having a diameter of 4.5 mm, is 16mm$^2$. This compares to a total cross-sectional of 7 mm$^2$ for vent design I in Fig. 17 (single base air inlet opening) and 14 mm$^2$ for the vent design II in Fig. 17 (dual base air inlet opening).

Fig. 24 shows the particle characteristics of the BDP (100/50) extrafine (EF) formulation as measured by FSA. The dose characteristics are obtained with vent designs I and II at a 12 mm base height and are compared with an actuator having a single air inlet opening in the base, with the air inlet opening having a diameter of 4.5 mm. The number of measurements was respectively n=3 for each actuator configuration.

The decrease in fine particle dose and extrafine particle dose between vent design I having one air inlet opening (see Fig. 17) and vent design II having two air inlet openings (see Fig. 17) is noticeable. However, if the cause of this decrease was due to the reduction in device resistance (~4kPa to ~1kPa) or the increased total cross-sectional area (from 7 mm$^2$ to 14 mm$^2$) associated with the different configurations, then a similar reduction would be expected to occur when using the single air inlet opening of 4.5 mm diameter in the actuator base (~1kPa and 16mm$^2$). Since there is little difference in the dose characteristics between a single 3.0 mm base air inlet opening (vent design I) and a single 4.5 mm base air inlet opening, the effect may not be attributable to total cross-sectional area or device resistance.

(c) Actuator having 32 mm base height and various vent designs, with air inlet opening located in actuator base

Among vent designs I-IV and for base heights of 12 mm, 32 mm and 52 mm, the greatest respirable dose was achieved with an actuator having a base height of 32 mm with two 3.0 mm diameter air inlet openings formed in the actuator base (vent design II in Fig 17), for all three formulations (see Fig. 20A-20C). Increasing the diameter of the single air inlet opening at 12 mm base height had a small effect on the respirable dose despite an increase in total cross-sectional area and a decrease in device resistance.

To confirm this, the diameter of the air inlet openings of the vent design having two air inlet openings in the actuator base was altered between 2.0 mm and 3.5 mm with 0.5 mm intervals.

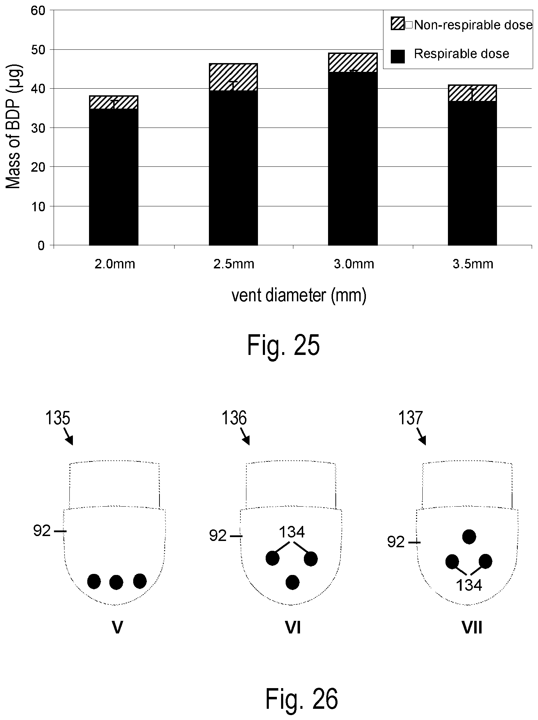

Fig. 25 shows the aerosol performance of BDP (100/50) extrafine (EF) formulation in response to increasing air inlet opening diameter at 32 mm base height. A total number of n=3 measurements were performed for each actuator configuration.

Interestingly, there is an increase in the respirable dose as the air inlet opening diameter increases up to 3.0 mm, after which performance drops (Fig. 25). These observations suggest an optimum diameter of 3.0 mm, among the different diameters tested. The calculated mean air velocity through an actuator having such a configuration is 33.4 m s$^{-1}$ (Table 3).

To investigate whether this value of mean air velocity represents an optimum velocity, a range of prototypes were designed which respectively have a base height of 32 mm, to match the velocity and total cross-sectional area based on vent design. The configurations of the air inlet openings manufactured using an air inlet opening diameter of 2.5 mm are illustrated in Fig. 26.

Design V, shown at 135, has three air inlet openings located in the base of the actuator. The air inlet openings have a linear arrangement. The diameter of each air inlet opening is 2.5 mm.

Design VI, shown at 136, and design VII, shown at 137, respectively have a triangular arrangement of air inlet openings. For both triangular arrangements, the positions of the two air inlet openings denoted at 134 in Fig. 26 are the same as for vent design II in Fig. 17, albeit with a lower diameter of 2.5 mm as compared to 3.0 mm. Design VI defines a "rear" triangle, pointing towards the actuator rear, and design VII defines a "front" triangle pointing towards the mouthpiece opening of the actuator.

All air inlet openings in designs V, VI and VII respectively have a diameter of 2.5 mm. The vent designs are distinguished in terms of the relative arrangement of the air inlet openings. The actual total cross-sectional area of the actuators is 14.7 mm$^2$ and the calculated mean air velocity at the air inlet openings is 32.0 m s$^{-1}$, which is comparable to the values for vent design II (Table 3).

The aerosol performance of the BDP (100/50) extrafine (EF) formulation was determined using the air inlet opening configurations of Fig. 26, to assess whether calculated mean air velocity and total cross-sectional area caused the "optimised" respirable dose obtained by the dual air inlet opening.

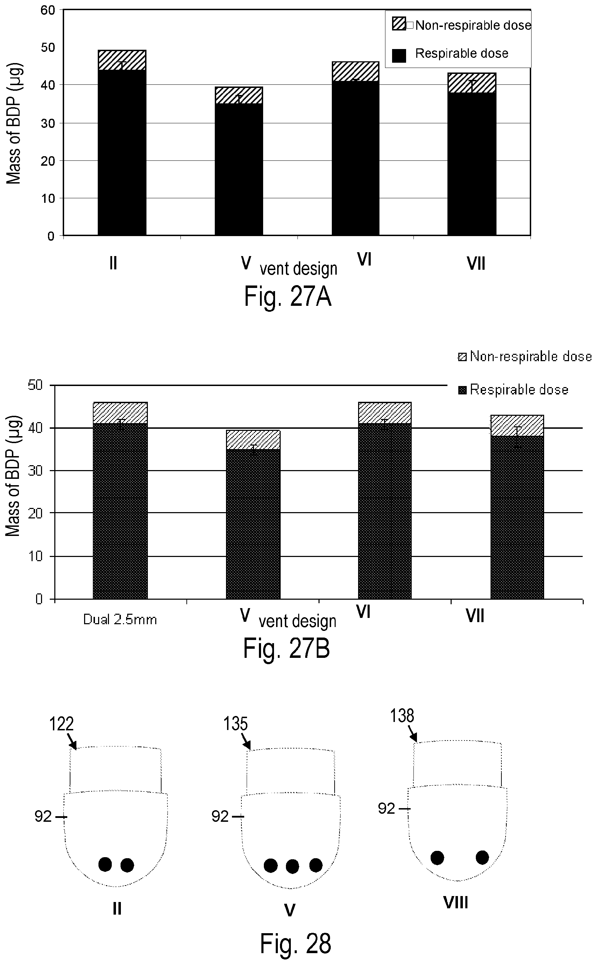

Fig. 27A shows the aerosol performance of the BDP (100/50) extrafine (EF) formulation in response to the designs with three air inlet openings using an air inlet opening diameter of 2.5 mm (designs V-VII in Fig. 26) compared to the dual air inlet opening design (design II in Fig. 17, having 3.0 mm diameter openings) at 32 mm base height. A total of n=3 measurements was performed. The data show the average ±SD.

The respirable dose obtained from the configurations with three air inlet openings was lower than that of the configurations having two air inlet openings (design II in Fig. 17). However, there was a slight difference between the actuators having three air inlet openings. The "rear" triangle configuration (design VI) out-performed the linear (design V) and front triangle (design VII) designs.

Fig. 27B shows the aerosol performance when the designs having three air inlet openings are compared with the designs having two air inlet openings of the same diameter, i.e. with a design having two air inlet openings of diameter 2.5 mm. In Fig. 27B, the aerosol performance of the BDP (100/50) extrafine formulation is shown in response to designs using an air inlet opening diameter of 2.5 mm at a base height of 32mm. A total of n=3 measurements was performed. The data show the average ±SD.

Interestingly, when the performances achieved with the designs having three air inlet openings are compared with the designs having two air inlet openings of the same vent diameter, the design with two air inlet openings having an air inlet opening diameter of 2.5 mm and the triple rear triangle design (design VI in Fig. 26) having an air inlet diameter of 2.5 mm are near identical. Furthermore, the front triangle design (design VII in Fig. 26) is only slightly less efficient. This suggests that it is the positioning and size of the air inlet openings indicated by 134 in Fig. 26 that contribute most to the respirable dose obtained, with the third air inlet opening causing a minimal effect.

The linear design with three air inlet openings (design V in Fig. 26) may deliver the lowest respirable dose since none of the air inlet opening positions match the design with two air inlet openings (see data in Fig. 27B).

To determine the significance of the two air inlet positions for the configuration with two air inlet openings, a further prototype was manufactured with an increased spacing between the air inlet openings. The different configurations are shown in Fig. 28. Configuration II, shown at 122, and configuration V, shown at 135, were already explained with reference to Figs. 17 and 26.

Design VIII, shown at 138, has two air inlet openings located in the base of the actuator. The diameter of each air inlet opening is 3 mm. The distance between the centers of the air inlet openings in design VIII is 10 mm, i.e., twice the distance of design II.

The position of the air inlet openings in design VIII, spaced 10 mm apart from each other relative to the centre, matches that of the outer air inlet openings used in vent design III or in vent design V (triple linear vent, see Figs. 17 and 26).

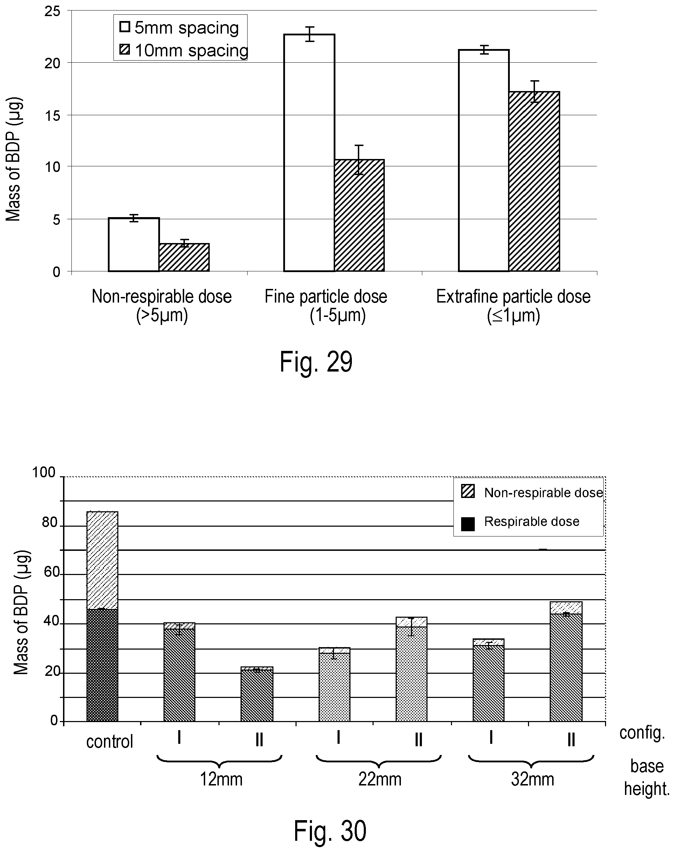

Fig. 29 shows the aerosol performance of the BDP (100/50) extrafine (EF) formulation when using vent design II (dual base air inlet openings, with 3.0 mm diameter and spacing 5 mm) and when using vent design VIII (dual base air inlet openings, with 3.0 mm diameter and spacing 10 mm).

By increasing the distance between the two air inlet openings, the performance has been drastically reduced, with an overall 37% reduction in respirable dose (from 44$\mu$g to 28$\mu$g). Furthermore, the reduction in respirable dose is mostly due to the reduction in the fine particle dose (1-5$\mu$m) and not the extrafine particle dose (<1$\mu$m).

(d) Vent designs showing best performance for actuators having base heights of 12 mm, 22 mm and 32 mm

For all formulations, vent design I (single air inlet opening in base, i.e. single base air inlet opening) and vent design II (two air inlet openings in base, i.e. dual base air inlet opening) produced the greatest respirable dose at base heights of 12 mm and 32 mm base respectively (Figures 20A-20C).

Further studies have revealed that the position of the air inlet openings has a significant effect on the respirable dose. The effect of the position may relate to the propagation of the plume over distance. For example, the characteristics of the plume in terms of droplet size, particle velocity and expansion will be different at a base height of 12 mm compared to a base height of 32 mm. Hence, a single air inlet opening may produce a dominant contribution, in terms of producing a high respirable dose, at a base height of 12 mm since it is focused on a specific region of the plume. As this region changes with distance, at a base height of 32 mm, a design having two air inlet openings in the base may produce a dominant contribution, in terms of producing a high respirable dose.

To determine which arrangement and configuration of air inlet openings produces a high respirable dose for an in-line actuator having a base height of 22 mm, two prototype in-line actuators were manufactured having a base height of 22 mm. The two actuators have the vent designs I and II shown in Fig. 17. The aerosol performance of the BDP (100/50) extrafine (EF) formulation at a base height of 22 mm with vent design I and vent design II is compared with the performance of actuators having base heights of 12 mm and 32 mm in Fig. 30.

Fig. 30 shows the aerosol performance of BDP (100/50) extrafine (EF) formulation using actuators having vent design I (single air inlet opening) and vent design II (dual air inlet openings) at base heights of 12 mm, 22 mm, and 32 mm (indicated as average for n=3 measurements, ±SD). The performance is compared to a conventional actuator with an orifice having a diameter of 0.22 mm (n=3; ±SD).

For a base height of 22 mm, the respirable dose is greater when using vent design II as compared to using vent design I.

The difference in performance between actuators having base heights of 22 mm and 32 mm when using vent design II is 5.2$\mu$g (Table 4). This difference is largely accounted for by a reduction in the proportion of fine particle dose $\leq$5$\mu$m and >1 $\mu$m, whereas the extrafine dose $\leq$1$\mu$m remains within one standard deviation. Conversely, between actuators having base heights of 12 mm and 22 mm, the difference in respirable dose is minimal. However, there is an increase in the proportion of extrafine particles compared to fine particles that contribute to the respirable dose. All respirable doses achieved by the in-line prototypes were within ±25% of the conventional MDI. The amount and fraction of non-respirable particles is significantly reduced compared to the conventional MDI.

Table 4: Dose characteristics of the BDP (100/50) extrafine (EF) formulation using vent design I at 12mm base height, and vent design II at 22mm and 32mm base height. The results are compared with a conventional MDI (number of measurements: n=3; average ±SD)

| Dose characteristics ($\mu$g) | Conventional, standard 0.22mm actuator | 12 mm base height- Design I | 22 mm base height - Design II | 32 mm base height - Design II |
|---|---|---|---|---|
| Metered dose | 95.9 (2.7) | 97.8 (3.7) | 93.5 (3.3) | 104.1 (0.1) |
| Delivered dose | 85.9 (3.2) | 40.3 (2.8) | 42.4 (3.1) | 49.0 (0.2) |
| Non-respirable dose (>5$\mu$m) | 39.9 (3.1) | 2.7 (1.1) | 3.7 (0.5) | 5.1 (0.4) |
| Respirable dose ($\leq$5$\mu$m) | 46.0 (0.5) | 37.6 (2.0) | 38.7 (3.4) | 43.9 (0.5) |
| Fine particle dose ($\leq$5 $\mu$m and >1$\mu$m) | 24.8 (0.9) | 11.7 (1.8) | 18.9 (0.7) | 22.7 (0.7) |
| Extrafine dose ($\leq$1$\mu$m) | 21.3 (1.4) | 26.0 (0.3) | 19.8 (2.8) | 21.2 (0.4) |

(d) Summary

At 12 mm base height, increasing and decreasing the diameter of the single air inlet opening (arranged as shown for vent design I in Fig. 1) did not affect the respirable dose obtained in the in-line design of an embodiment for the diameters tested.

At 32 mm base height, increasing and decreasing the diameter of the air inlet openings in the dual vent design did affect respirable dose. Among the various diameters tested, a diameter of 3.0 mm produced the best performance.

The performance obtained when using the configuration with two air inlet openings in the base, each having a diameter of 3.0 mm, was not related to cross-sectional area or calculated mean air velocity, but was highly dependent on positioning.

Air velocity plays a role in producing the observed respirable dose, but is not as critical as position of the air inlet opening(s).

Between 12 mm and 32 mm, the configuration of air inlet openings that produces the best performance, among the different configurations tested, moves from the configuration having one air inlet opening to the configuration having two air inlet openings.

The propagation of the plume causes an increase in the expansion over increasing distance until a maximum is reached. During this expansion, droplet size and velocity are changing. This spray pattern within the actuator likely accounts for the observed effects.

ACTUATORS HAVING AT LEAST ONE AIR INLET OPENING IN AN ACTUATOR BASE AND AT LEAST ONE AIR INLET OPENING IN A REAR WALL

To investigate the effect of combined vent designs, two additional prototypes of actuators according to embodiments were manufactured. The actuators had a base height of 32 mm.

The actuators were provided with air inlet configurations, or vent designs, which had both air inlet openings located in the actuator base and an air inlet opening located in a rear wall. More specifically, the following vent designs were used:

Design IX had two air inlet openings of diameter 3.0 mm in the actuator base (positioned as shown in Fig. 17 for vent design II) in combination with one air inlet opening located in a rear wall of the actuator. The air inlet opening located in the rear wall had a diameter of 3.0 mm. The center of the air inlet opening located in the rear wall was positioned at 10 mm from the actuator base.

Design X had two air inlet openings of diameter 3.0 mm in the actuator base (positioned as shown in Fig. 17 for vent design II) in combination with one air inlet opening located in a rear wall of the actuator. The air inlet opening located in the rear wall had a diameter of 3.0 mm. The center of the air inlet opening located in the rear wall was positioned at 20 mm from the actuator base.

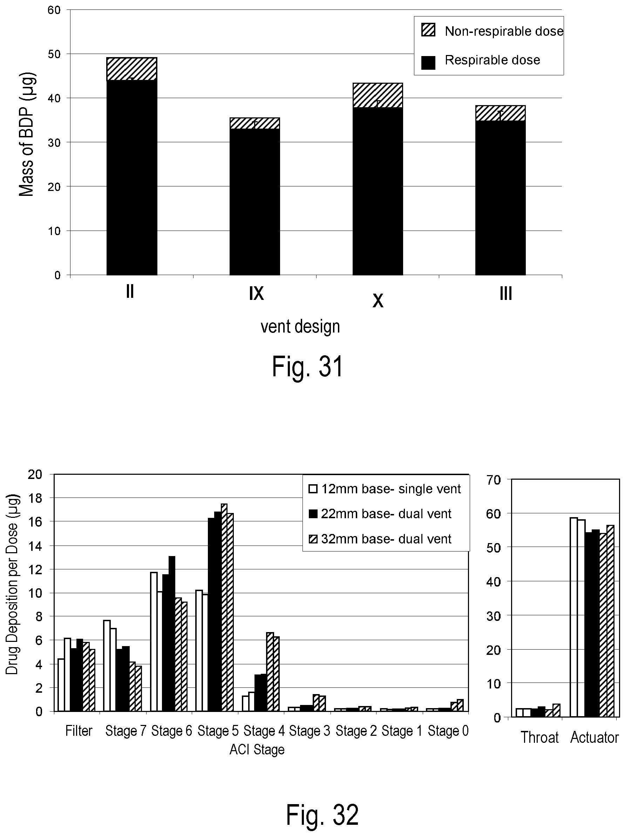

Fig. 31 shows the performance of the BDP (100/50) extrafine (EF) formulation using the combined base and rear vent designs IX and X, for a base height of 32 mm (averaged data for n=3 measurements $\pm$SD). The results are compared to base vent design II and III (see Fig. 17) at a base height of 32 mm (averaged data for n=3 measurements $\pm$SD).

When compared to each other, the vent design in which the rear air inlet opening is located closer to the orifice (i.e., at a height of 20 mm from the actuator base) produces a greater respirable dose. When the performance of these combined vent prototypes is compared to the original vent design III, in which the overall number of air inlet openings is the same, there is a slight increase in respirable dose, which may be attributed to the position of the third air inlet opening. However, the difference is small and does not compare to the performance achieved using vent design II.

ACI STUDIES

Based upon the optimisation studies in terms of air inlet opening configurations, configurations between 12 mm and 32 mm base height are able to produce a respirable dose within $\pm$25% of a conventional MDI. This section will focus on confirming the results obtained using the FSA with the ACI according to the methodology outlined above.

(a) ACI studies for actuators having base heights of 12 mm, 22 mm and 32 mm

The performance of the actuator configurations showing the best performance for base heights of 12 mm, 22 mm and 32 mm (vent design I for base height of 12 mm, vent design II for base height of 22 mm and for base height of 32 mm) with the three test formulations as measured using the ACI is given in Figs. 32-34. The air inlet openings were respectively provided in the actuator base.

Fig. 32 shows the data for the BDP (100/50) extrafine (EF) formulation, which were obtained using an actuator of an embodiment having a base height of 12 mm and vent design I, an actuator of an embodiment having a base height of 22 mm and vent design II, and an actuator of an embodiment having a base height of 32 mm and vent design II. Data of two measurements (n=2) for each one of the actuators are shown.

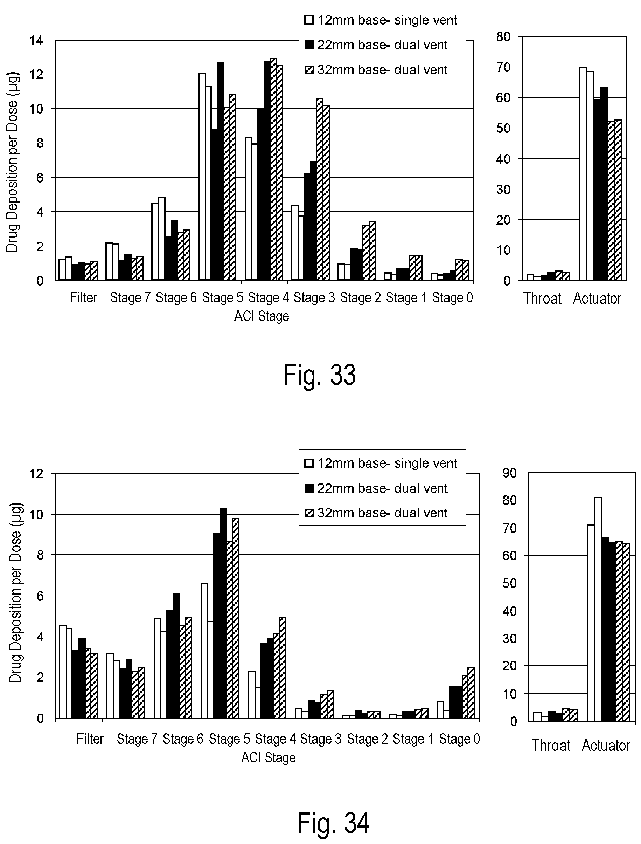

Fig. 33 shows the data for the BDP (100/50) low volatility component (LVC) formulation, which were obtained using an actuator of an embodiment having a base height of 12 mm and vent design I, an actuator of an embodiment having a base height of 22 mm and vent design II, and an actuator of an embodiment having a base height of 32 mm and vent design II. Data of two measurements (n=2) for each one of the actuators are shown.

Fig. 34 shows the data for the BDP (100/50) high ethanol (HE) content formulation, which were obtained using an actuator of an embodiment having a base height of 12 mm and vent design I, an actuator of an embodiment having a base height of 22 mm and vent design II, and an actuator of an embodiment having a base height of 32 mm and vent design II. Data of two measurements (n=2) for each one of the actuators are shown.

The dose characteristics for each formulation are given in Tables 5-7. For comparison, control data has been included for a conventional actuator having an orifice diameter of 0.22 mm and for a conventional actuator having an orifice diameter of 0.30 mm.

The aerosol performance as determined by ACI shows that the fine particle dose ($\leq 5\mu$m) achieved at 12 mm base height using the single air inlet opening design (vent design I in Fig. 17) is lower than the results obtained from the actuators having 22 mm and 32 mm base height and two air inlet openings (vent design II). This discrepancy between the FSA and ACI data may be due to the difference between the void volumes of the two impactors in combination with the use of a single vent design, which offers a higher resistance to air flow. The difference between the conventional actuator having an orifice diameter of 0.22 mm and the actuator of an embodiment having a base height of 12 mm is greatest for the extrafine formulation, achieving only 69.4% of the respirable dose of the conventional actuator (Table 5). This compares with 72.3% and 77.7% of the respirable dose of the conventional actuator with 0.22 mm orifice diameter achieved for the high ethanol (HE) and low volatility component (LVE) formulations respectively.

For actuators having base heights of 22 mm and 32 mm, fine particle doses are achieved that are well within $\pm$25% of the conventional actuator having an orifice diameter of 0.22 mm.

Data has also been obtained for the performance of the BDP (100/50) extrafine (EF) formulation and low volatility component (LVC) formulation in a conventional actuator having an orifice diameter of 0.30 mm actuator (Tables 5 and 6). In both instances, the actuator of an embodiment having an in-line configuration out-performs the conventional actuator with 0.30 mm orifice diameter in terms of fine particle dose, particularly at base heights of 22 mm and 32 mm. At a base height of 12 mm, the data is comparable, although the mass median aerodynamic diameter (MMAD) achieved using the in-line actuator of an embodiment is still lower than that achieved by the conventional actuator.

Table 5: Dose characteristics for the BDP (100/50) extrafine (EF) formulation using the actuators according to embodiments at three base heights (n=2 measurements). Aerosol performance of a conventional actuator having an orifice diameter of 0.20 mm (average; n=3 $\pm$SD) and of a conventional actuator having an orifice diameter of 0.30 mm (average obtained for n=2 measurements) is included.

| | 12mm height | base | 22mm height | base | 32mm height | base | Control 0.22mm | Control 0.30mm |
|---|---|---|---|---|---|---|---|---|
| Metered Dose ($\mu$g) | 96.8 | 95.6 | 99.2 | 103.8 | 102.6 | 104.2 | 98.2 $\pm$ 1.8 | 95.9 |
| Delivered Dose ($\mu$g) | 38.3 | 37.7 | 44.9 | 48.6 | 48.5 | 47.8 | 88.2 $\pm$ 2.5 | 86.7 |
| FPD ($\mu$g) | 35.6 | 34.9 | 42.0 | 45.1 | 45.1 | 42.5 | 50.8 $\pm$ 3.1 | 33.0 |
| FPF (%) | 93.0 | 92.7 | 93.4 | 92.7 | 92.9 | 88.9 | 57.5 $\pm$ 2.0 | 38.1 |
| MMAD ($\mu$m) | 0.9 | 0.9 | 1.1 | 1.0 | 1.2 | 1.3 | 1.3 $\pm$ 0.0 | 1.5 |
| GSD | 1.8 | 1.8 | 2.1 | 2.1 | 2.4 | 2.3 | 2.1 $\pm$ 0.0 | 2.4 |
| Shot Weight (mg) | 56.4 | 55.0 | 56.2 | 57.3 | 56.5 | 57.9 | 55.2 $\pm$ 0.3 | 53.5 |

For the actuators of embodiments, the orifice diameter of the prototype actuator is 0.26 mm, precisely halfway between the nozzle orifice diameters of 0.22 mm and 0.30 mm of the conventional actuators. Therefore the performance of the prototype is in line with orifice diameter.

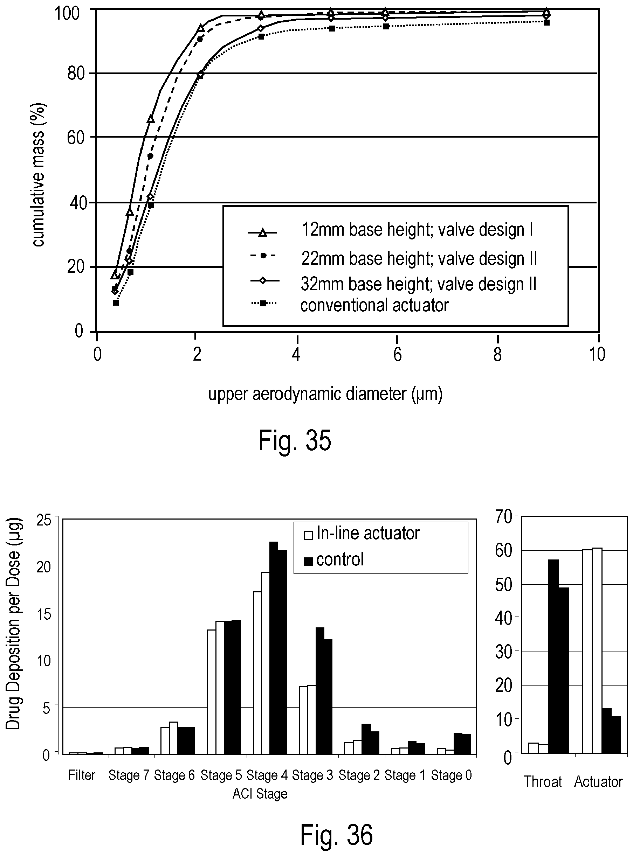

Fig. 35 shows the cumulative mass undersize of the BDP (100/50) extrafine (EF) formulation using an actuator of an embodiment having a base height of 12 mm and vent design I, an actuator of an embodiment having a base height of 22 mm and vent design II, and an actuator of an embodiment having a base height of 32 mm and vent design II. For comparison, data obtained with a conventional actuator having an orifice diameter of 0.22 mm are also shown (average for n=3 measurements).

Interestingly, an increase in the base height of the in-line actuator causes an upward shift in the MMAD of the formulation, and gradually approaches that of the conventional actuator with 0.22 mm orifice diameter. Therefore, the resultant particle size distribution of the formulation may be altered by selecting an appropriate optimised base height.

As the base height increases, the MMAD (Table 5) and cumulative mass (%) undersize approach that of the conventional actuator having an orifice diameter of 0.22 mm. The magnitude of the shift in MMAD is greatest in the low volatility component (LVC) formulation (Table 6), with a rise of 0.9 $\mu$m as base height increases from 12 mm to 32 mm. For the high ethanol (HE) content formulation and the extrafine (EF) formulation, the increase was 0.5 $\mu$m and 0.4 $\mu$m respectively. This difference may be related to the amount of non-volatile content (NVC) within each formulation. The inclusion of glycerol in the low volatility component (LVC) formulation increases the NVC from 0.175%w/w to 1.475%w/w as compared to the extrafine (EF) formulation and high ethanol (HE) content formulation. The contribution of the upper particle sizes for the calculated values of MMAD is therefore greater. Hence the removal of large particle sizes induced by the actuator has a greater effect on MMAD.

Table 6: Dose characteristics for the BDP (100/50) with low volatility component (LVC) formulation using the actuators according to embodiments at three base heights (n=2 measurements). Aerosol performance of a conventional actuator having an orifice diameter of 0.20 mm (average for n=3 $\pm$SD) and of a conventional actuator having an orifice diameter of 0.30 mm (average for n=3) is included.

| | 12mm height | base | 22mm height | base | 32mm height | base | Control 0.22mm | Control 0.30mm |
|---|---|---|---|---|---|---|---|---|
| Metered Dose ($\mu$g) | 106.2 | 102.8 | 93.4 | 107.4 | 99.5 | 100.1 | 95.4 $\pm$ 1.4 | 99.1 |
| Delivered Dose ($\mu$g) | 36.3 | 34.2 | 34.1 | 44.1 | 47.5 | 47.6 | 85.5 $\pm$ 1.8 | 89.1 |

(continued)

|  | 12mm height | base | 22mm height | base | 32mm height | base | Control 0.22mm | Control 0.30mm |
|---|---|---|---|---|---|---|---|---|
| FPD ($\mu$g) | 32.7 | 31.5 | 30.3 | 39.0 | 39.6 | 40.0 | 41.4 ± 2.1 | 26.2 |
| FPF (%) | 90.3 | 92.2 | 88.9 | 88.5 | 83.5 | 84.1 | 48.4 ± 3.0 | 29.4 |
| MMAD ($\mu$m) | 1.8 | 1.8 | 2.4 | 2.2 | 2.7 | 2.6 | 2.8 ± 0.2 | 3.3 |
| GSD | 2.0 | 2.1 | 2.0 | 2.0 | 2.1 | 2.1 | 2.2 ± 0.1 | 2.4 |
| Shot Weight (mg) | 56.5 | 56.0 | 54.1 | 56.3 | 55.1 | 56.8 | 56.2 ± 0.4 | 54.7 |

Table 7: Dose characteristics for the BDP (100/50) high ethanol (HE) content formulation using the actuators according to embodiments at three base heights (n=2 measurements). Aerosol performance of a conventional actuator having an orifice diameter of 0.20 mm (average; n=3 ±SD) is included.

|  | 12mm height | base | 22mm height | base | 32mm height | base | Control 0.22mm |
|---|---|---|---|---|---|---|---|
| Metered Dose ($\mu$g) | 97.2 | 101.6 | 96.6 | 97.3 | 96.5 | 98.5 | 96.5 ± 1.5 |
| Delivered Dose ($\mu$g) | 26.3 | 20.5 | 30.3 | 32.5 | 31.3 | 34.0 | 84.8 ± 1.9 |
| FPD ($\mu$g) | 21.9 | 18.0 | 24.6 | 27.8 | 24.2 | 26.7 | 27.6 ± 1.5 |
| FPF (%) | 83.4 | 87.8 | 81.1 | 85.8 | 77.4 | 78.4 | 32.6 ± 1.2 |
| MMAD ($\mu$m) | 1.0 | 0.9 | 1.3 | 1.2 | 1.4 | 1.5 | 1.6 ± 0.1 |
| GSD | 2.2 | 2.2 | 2.4 | 2.5 | 2.7 | 2.4 | 2.4 |
| Shot Weight (mg) | 51.4 | 51.5 | 51.4 | 51.7 | 51.9 | 51.5 | 50.6 ± 0.6 |

(b) Effect of non-volatile content

To determine the effect of increasing non-volatile content on the performance of the actuator compared to a conventional actuator, additional tests were performed for a prototype of an actuator according to an embodiment, having a base height of 32 mm and vent design II (Fig. 17). Ideally, the performance of an in-line actuator which has a suitably selected base height and/or vent design, or of an in-line actuator which is optimized with regard to base height and vent design, would be only weakly affected or essentially unaffected by any differences in formulation. As shown above, increasing the non-volatile content (NVC) in the formulations (e.g. formulation with the low volatility component, LVC, compared to extrafine formulation, EF, and high ethanol content formulation, HE) resulted in an enhanced effect on the upward shift of MMAD as base height increases.

The effect of increasing the non-volatile content was assessed for an actuator according to an embodiment, having a base height of 32 mm and vent design II (Fig. 17). Additional BDP formulations "High NVC" and "Low NVC" as specified in Table 1 were prepared. The packaging for the formulations was as stated above. The low volatility component (LVC) formulation and the extrafine (EF) formulation were used for comparison, giving an overall range of NVC from 0.01%w/w to 1.475%w/w (Table 8). Delivery characteristics of each formulation were tested in the actuator of an embodiment with 32 mm base height and a conventional actuator having an orifice diameter of 0.22 mm. The results and comparisons are given in Table 8.

Table 8: Comparison of dose and particle size distribution between BDP formulations containing a range of non-volatile content with 13%w/w ethanol (average; n=2 for BDP (6/50) and BDP (250/50); n=3 for BDP (100/50) and BDP (100/50))

| | BDP (6/50) | BDP (100/50) EF formulation | BDP (250/50) | BDP (100/50) LVC formulation |
|---|---|---|---|---|
| NVC (%w/w) | 0.010 | 0.175 | 0.438 | 1.475 |
| Embodiment: | | | | |
| FPD | 2.2 | 43.8 | 97.9 | 39.8 |
| MMAD | 0.7 | 1.3 | 1.6 | 2.7 |
| Conventional: | | | | |
| FPD | 3.0 | 50.8 | 114.6 | 41.3 |
| MMAD | 0.7 | 1.3 | 1.8 | 2.8 |
| % FPD of conventional | 73.3% | 86.2% | 85.4% | 96.6% |
| MMAD difference | 0.0 | 0.0 | 0.2 | 0.1 |

With increasing non-volatile content, the match between the fine particle dose ($\leq 5\mu$m) achieved using the in-line actuator with a base height of 32 mm and the conventional actuator having an orifice diameter of 0.22 mm improves. Although there is a slightly reduced value for the MMAD between the formulations as the non-volatile content is increased, the difference in small. This demonstrates that the optimised in-line design at 32 mm base height can achieve the same particle size distribution as the conventional 0.22 mm actuator.

(c) Suspension formulation containing ethanol

To assess the efficiency of the actuator of an embodiment, having an in-line configuration, with the use of a suspension formulation, a model product containing salbutamol sulphate (Salamol® IVAX) was selected.

One metered dose contains salbutamol sulphate equivalent to 100 micrograms salbutamol;
Excipients:

    ethanol, anhydrous
    Norflurane (propellant HFA-134a).

The formulation is contained in a pressurised aluminium container with a metering valve.

The conventional actuator provided with Salamol® is a breath-activated device. To assess the performance of the product, the conventional actuator was opened and hand-actuated, thus serving as a control. For the actuator of an embodiment, the canister removed from the Salamol® device was placed in the prototype of an actuator of an embodiment, having a base height of 32 mm and two air inlet openings formed in the actuator base (vent design II in Fig. 17), to assess performance. The size of the orifice used on the control device, as measured using optical stereo microscopy (Nikon SM2800), is 0.24 mm. This is directly comparable to the orifice diameter of the in-line actuator according to an embodiment, where the orifice diameter is 0.26 mm. Therefore, any differences between the aerosol performance of the formulation are unlikely to be due to orifice diameter.

Fig. 36 shows the aerosol performance (particle size distribution) of ($100\mu$g/$25\mu$L) (100 $\mu$g as salbutamol; 117.01 $\mu$g as salbutamol sulphate) suspended salbutamol sulphate using the prototype for the in-line actuator with base height 32 mm and vent design II, and of the control device. Two measurements were performed for each device (n=2).

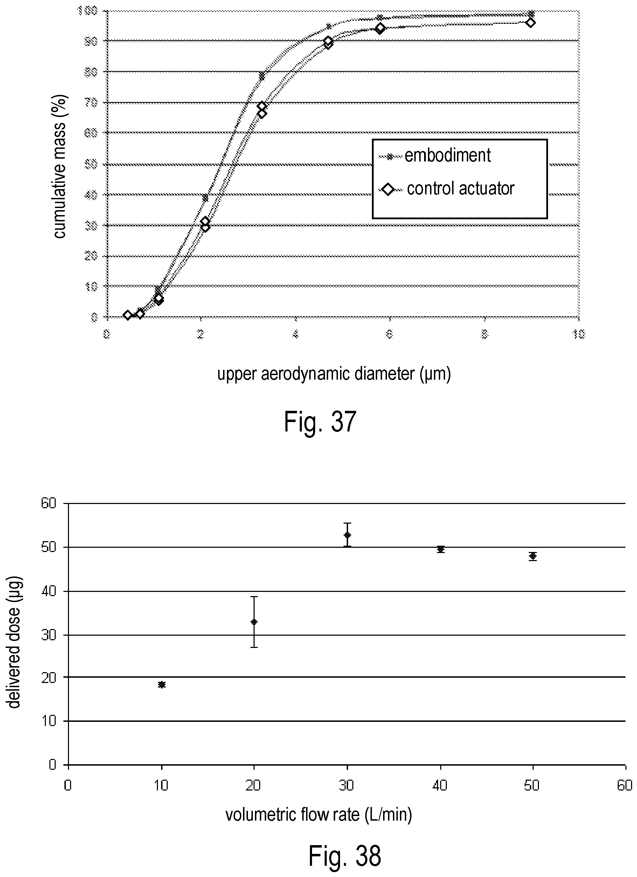

Fig. 37 shows the cumulative mass undersize of salbutamol sulphate (100/25) using the in-line actuator with 32 mm base height and the control device. Data obtained in two measurements (n=2) are shown for each device.

Table 9 shows the dose characteristics.

The comparison between the control device and the prototype of an in-line actuator showed similar deposition profiles up to stage 5. Above this, the control device delivers a slightly higher dose (see also the very high deposition into the throat).

Table 9: Dose characteristics for salbutamol sulphate (100/25) (actual dose: 117.01$\mu$g salbutamol sulphate) using the in-line actuator with 32 mm base height and the control device (n=2 measurements)

| | In-line with base height | actuator 32mm | | control |
|---|---|---|---|---|
| Metered Dose ($\mu$g) | 107.2 | 111.0 | 130.9 | 117.2 |
| Delivered Dose($\mu$g) | 46.8 | 50.3 | 117.6 | 106.2 |
| FPD ($\mu$g) | 41.7 | 45.6 | 54.6 | 52.5 |
| FPF (%) | 89.1 | 90.6 | 46.4 | 49.5 |
| MMAD ($\mu$m) | 2.4 | 2.4 | 2.7 | 2.6 |
| GSD | 1.7 | 1.7 | 1.7 | 1.7 |
| Shot Weight (mg) | 33.5 | 33.8 | 34.6 | 33.5 |

The percentage difference between the average fine particle dose achieved from the actuator of an embodiment compared to the control device is 81.5%. This difference is comparable to that found for the extrafine (EF) formulation (Table 8 above). Based on the cumulative undersize there is a shift in MMAD between the in-line actuator and the control device (Fig. 37) which is slightly greater than that observed with solution formulations for an in-line actuator having a base height of 32 mm. This is probably due to the slight differences in behaviour of a suspended and solution formulation.

FLOW RATE DEPENDENCY

For actuators of embodiments, operation relies upon the inspiratory effort of the patient to produce airflow through the in-line actuator. The experimental data described above has been obtained for a flow rate of 28.3 ($\pm$5%) L min$^{-1}$, as per the standard testing requirements for a MDI system. However, as air flow within the device determines the respirable dose, it is desirable to evaluate how the performance depends on the inspiratory flow rate. Particle size analysis using the FSA and ACI impactors relies upon careful calibration at a single flow rate, rendering them unsuitable for use at different flow rates. Therefore the flow rate dependency has been evaluated by examining the differences in delivered dose using a sample collection tube over a range of flow rates from 10 L min$^{-1}$ to 50 L min$^{-1}$, in steps of 10 L min$^{-1}$. Since the actuator according to embodiments produces a high fine particle fraction due to the removal of the larger particles through device design, measurement of the delivered dose will be an accurate reflection of the performance.

Tests were performed for a prototype of an actuator of an embodiment, having a base height of 32 mm and two air inlet openings (vent design II in Fig. 17) located in the base.

The delivered dose achieved at a range of flow rates is given in Fig. 38 and the resultant actuator deposition in Fig. 39. Fig. 38 shows the delivered dose from the BDP (100/50) extrafine (EF) formulation using the prototype of an actuator according to an embodiment, with a base height of 32 mm (data show average obtained for n=4 measurements $\pm$SD). Fig. 39 shows the average of actuator deposition from BDP (100/50) extrafine (EF) formulation in response to a range of volumetric flow rates. Data show the average of 5 shots for the prototype of an actuator according to an embodiment, with a base height of 32 mm and vent design II (Fig. 17).

As the flow rate increases up to 30 L min$^{-1}$, there is a significant dependency of device performance on flow rate. However, after 30 L min$^{-1}$ the increase in performance does not continue and the response reaches a plateau. There is likely to be a loss of dose if a patient does not achieve a flow rate of approximately 30 L min$^{-1}$, but the use of a stronger flow rate should not result in a higher dose.

**Claims**

1. A metered-dose inhaler actuator, comprising:

   a housing having a mouthpiece portion (13) and a canister receiving portion (12) configured to receive a canister (2), said housing extending from an opening (21) for receiving said canister (2) to a mouthpiece opening (22);
   a member (14) disposed within said housing and defining a valve stem receptacle (15) configured to receive a valve stem (3) of said canister (2), an orifice (16; 73, 74; 76, 77; 79, 80; 84; 88) for emitting an aerosol plume being formed in said member (14), said orifice (16; 73, 74; 76, 77; 79, 80; 84; 88) being in fluid communication with said valve stem receptacle (15) and extending to a face (19) of said member (14) opposite to said valve stem receptacle (15);
   a longitudinal axis (18) of said orifice (16; 73, 74; 76, 77; 79, 80; 84; 88) being aligned with a longitudinal axis (17) of said valve stem receptacle (15); and

at least one air inlet opening (20) being provided in an outer shell of said housing so as to be spaced from said opening (21) for receiving said canister (2) and from said mouthpiece opening (22), said at least one air inlet opening (20) being in fluid communication with said mouthpiece opening (22), and

a longitudinal axis (25) of said mouthpiece portion (13) being arranged at an angle and not parallel relative to said longitudinal axis (18) of said orifice (16; 73, 74; 76, 77; 79, 80; 84; 88),

**characterized in that**

an air inlet opening (20) of said at least one air inlet opening (20) is arranged to produce an airflow essentially perpendicular to the direction of the aerosol plume and is positioned on a line of view (29) which passes through said mouthpiece opening (22) and is aligned with said longitudinal axis (25) of said mouthpiece portion (13), and is arranged to produce an airflow essentially perpendicular to the direction of the aerosol plume.

2. The actuator of claim 1,
said at least one air inlet opening (20) being provided in a part of said outer shell of said housing extending from said member (14) towards said mouthpiece opening (22).

3. The actuator of claim 1 or claim 2,
said housing including a wall oriented at an angle relative to said longitudinal axis (25) of said mouthpiece portion (13), an air inlet opening (20) of said at least one air inlet opening (20) being provided in said wall.

4. The actuator of any one of the preceding claims,
each air inlet opening (20) of said at least one air inlet opening (20) being respectively positioned on a line of view (29) which passes through said mouthpiece opening (22) and is aligned with said longitudinal axis (25) of said mouthpiece portion (13).

5. The actuator of any one of the preceding claims,
said member (14) and said air inlet opening (20) being configured such that, in use of the actuator (11; 31; 41; 51; 91), all air output via said mouthpiece opening (22) is drawn into an interior of said housing through said at least one air inlet opening (20).

6. The actuator of any one of the preceding claims,
said member (14) extending across a cross sectional area of said canister receiving portion (12).

7. The actuator of claim 6,
said member (14) being configured to block passage of gas past said member (14) radially outwardly of said orifice (16; 73, 74; 76, 77; 79, 80; 84; 88).

8. The actuator of any one of the preceding claims,
said orifice (73, 74; 76, 77; 79, 80) having at least a portion (73; 76; 79) tapering towards said face (19) of said member (14) opposite to said valve stem receptacle (15).

9. The actuator of claim 8,
said tapering portion (79) of said orifice (79, 80) having a maximum diameter corresponding to an outer diameter of said valve stem (3).

10. The actuator of claim 8,
said tapering portion (76) of said orifice (76, 77) having a maximum diameter corresponding to an inner diameter of said valve stem (3).

11. The actuator of any one of the preceding claims,
an expansion chamber (82, 83; 86, 87) being formed in said member (14), said expansion chamber (82, 83; 86, 87) being in fluid communication with said orifice (84; 88) and said valve stem receptacle (15) and having a longitudinal axis aligned with said longitudinal axis (17) of said valve stem receptacle (15).

12. The actuator of any one of the preceding claims,
said longitudinal axis (18) of said orifice (16; 73, 74; 76, 77; 79, 80; 84) being disposed at an angle (28) of greater than 90° relative to said longitudinal axis (25) of said mouthpiece portion (13).

13. A metered-dose inhaler, comprising
the actuator (11; 31; 41; 51; 91) of any one of the preceding claims, and
a canister (2) provided with a metering valve (32) which comprises a valve stem (3) to be fitted into said valve stem receptacle (15) formed in said member (14) of said actuator (11; 31; 41; 51), said canister (2) containing an aerosol formulation.

14. Non therapeutic use of an actuator (11; 31; 41; 51; 91) according to any one of claims 1-12 for dispensing an aerosol formulation from a canister (2).

**Patentansprüche**

1. Dosierinhalator-Aktuator, umfassend:

ein Gehäuse, das einen Mundstückteil (13) und einen Kartuschenaufnahmeteil (12), der zum Aufnehmen einer Kartusche (2) konfiguriert ist, hat, wobei sich das Gehäuse von einer Öffnung (21) zum Aufnehmen der Kartusche (2) zu einer Mundstücköffnung (22) erstreckt;
ein Element (14), angeordnet in dem Gehäuse und definierend ein Ventilschaftbehältnis (15), das konfiguriert ist, um einen Ventilschaft (3) der Kartusche (2) aufzunehmen, eine Düse (16; 73, 74; 76, 77; 79, 80; 84; 88) zum Ausstoßen einer Aerosolfahne, die in dem Element (14) gebildet wird, wobei die Düse (16; 73, 74; 76, 77; 79, 80; 84; 88) in Fluidkommunikation mit dem Ventilschaftbehältnis (15) ist und sich zu einer Fläche (19) des Elementes (14) erstreckt, die dem Ventilschaftbehältnis (15) gegenüberliegt;
eine Längsachse (18) der Düse (16; 73, 74; 76, 77; 79, 80; 84; 88), die mit einer Längsachse (17) des Ventilschaftbehältnisses (15) ausgerichtet ist; und
wenigstens eine Lufteinlassöffnung (20), die in einer Außenschale des Gehäuses so angeordnet ist, dass sie von der Öffnung (21) zum Aufnehmen der Kartusche (2) und von der Mundstücköffnung (22) beabstandet ist, wobei die wenigstens eine Lufteinlassöffnung (20) in Fluidkommunikation mit der Mundstücköffnung (22) ist, und eine Längsachse (25) des Mundstückteils (13), die in einem Winkel und nicht parallel bezüglich der Längsachse (18) der Düse (16; 73, 74; 76, 77; 79, 80; 84; 88) angeordnet ist;
**dadurch gekennzeichnet, dass**
eine Lufteinlassöffnung (20) der wenigstens einen Lufteinlassöffnung (20) angeordnet ist, um einen Luftstrom im Wesentlichen senkrecht zu der Richtung der Aerosolfahne produzieren, und auf einer Blicklinie (29) positioniert ist, welche durch die Mundstücköffnung (22) geht und mit der Längsachse (25) des Mundstückteils (13) ausgerichtet ist und angeordnet ist, um einen Luftstrom im Wesentlichen senkrecht zu der Richtung der Aerosolfahne zu produzieren.

2. Aktuator gemäß Anspruch 1,
wobei wenigstens eine Lufteinlassöffnung (20) in einem Teil der Außenschale des Gehäuses angeordnet ist, der sich von dem Element (14) in Richtung der Mundstücköffnung (22) erstreckt.

3. Aktuator gemäß Anspruch 1 oder Anspruch 2,
wobei das Gehäuse eine Wand umfasst, die in einem Winkel bezüglich der Längsachse (25) des Mundstückteils (13) orientiert ist, eine Lufteinlassöffnung (20) der wenigstens einen Lufteinlassöffnung (20) in der Wand angeordnet ist.

4. Aktuator gemäß einem der vorangehenden Ansprüche,
wobei jede Lufteinlassöffnung (20) der wenigstens einen Lufteinlassöffnung (20) jeweils in einer Blicklinie (29) positioniert ist, welche durch die Mundstücköffnung (22) geht und mit der Längsachse (25) des Mundstückteils (13) ausgerichtet ist.

5. Aktuator gemäß einem der vorangehenden Ansprüche,
wobei das Element (14) und die Lufteinlassöffnung (20) so konfiguriert sind, dass bei Verwendung des Aktuators (11; 31; 41; 51; 91) der gesamte Luftausstoß über die Mundstücköffnung (22) in das Innere des Gehäuses durch die wenigstens eine Lufteinlassöffnung (20) gezogen wird.

6. Aktuator gemäß einem der vorangehenden Ansprüche,
wobei sich das Element (14) über einen Querschnittsbereich des Kartuschenaufnahmeteils (12) erstreckt.

**7.** Aktuator gemäß Anspruch 6,
wobei das Element (14) konfiguriert ist, um eine Passage von Gas nach dem Element (14) radial nach außen der Düse (16; 73, 74; 76, 77; 79, 80; 84; 88) zu blockieren.

**8.** Aktuator gemäß einem der vorangehenden Ansprüche,
wobei die Düse (73, 74; 76, 77; 79, 80) wenigstens einen Abschnitt (73; 76; 79) hat, der sich in Richtung der Fläche (19) des Elementes (14), das dem Ventilschaftaufnahmebehältnis (15) gegenüber liegt, konisch verjüngt.

**9.** Aktuator gemäß Anspruch 8,
wobei der sich konisch verjüngende Abschnitt (79) der Düse (79, 80) einen maximalen Durchmesser hat, der dem Außendurchmesser des Ventilschafts (3) entspricht.

**10.** Aktuator gemäß Anspruch 8,
wobei der sich konisch verjüngende Abschnitt (76) der Düse (76, 77) einen maximalen Durchmesser hat, der dem Innendurchmesser des Ventilschafts (3) entspricht.

**11.** Aktuator gemäß einem der vorangehenden Ansprüche,
wobei eine Expansionskammer (82, 83; 86, 87) in dem Element (14) gebildet ist, die Expansionskammer (82, 83; 86, 87) in Fluidkommunikation mit der Düse (84; 88) und dem Ventilschaftbehältnis (15) ist und eine Längsachse hat, die mit der Längsachse (17) des Ventilschaftaufnahmebehältnisses (15) ausgerichtet ist.

**12.** Aktuator gemäß einem der vorangehenden Ansprüche,
wobei die Längsachse (18) der Düse (16; 73, 74; 76, 77; 79, 80; 84) in einem Winkel (28) von größer als 90° bezüglich der Längsachse (25) des Mundstücksteils (13) angeordnet ist.

**13.** Dosierinhalator, umfassend
den Aktuator (11; 31; 41; 51; 91) gemäß einem der vorangehenden Ansprüche, und
einen Kartusche (2), die mit einem Dosierventil (32) versehen ist, das einen Ventilschaft (3) zur Einpassung in das Ventilschaftaufnahmebehältnis (15), das in dem Element (14) des Aktuators (11; 31; 41; 51) gebildet ist, umfasst,
wobei die Kartusche (2) eine Aerosolformulierung enthält.

**14.** Nicht-therapeutische Verwendung eines Aktuators (11; 31; 41; 51; 91) gemäß einem der Ansprüche 1-12 zum Abgeben einer Aerosolformulierung aus einer Kartusche (2).

**Revendications**

**1.** Actionneur d'inhalateur-doseur, comprenant :

un logement ayant une partie d'embout buccal (13) et une partie réceptrice de cartouche (12) conçue pour recevoir une cartouche (2), ledit logement s'étendant depuis une ouverture (21) destinée à recevoir ladite cartouche (2) jusqu'à une ouverture d'embout buccal (22) ;
un élément (14) disposé à l'intérieur du logement et définissant un réceptacle de tige de soupape (15) conçu pour recevoir une tige de soupape (3) de la cartouche (2), un orifice (16 ; 73, 74 ; 76, 77 ; 79, 80 ; 84 ; 88) pour émettre un nuage d'aérosol qui est produit dans ledit élément (14), ledit orifice (16 ; 73, 74 ; 76, 77 ; 79, 80 ; 84 ; 88) étant en communication fluidique avec le réceptacle de tige de soupape (15) et s'étendant vers une face (19) dudit élément (14) à l'opposé du réceptacle de tige de soupape (15) ;
un axe longitudinal (18) de l'orifice (16 ; 73, 74 ; 76, 77 ; 79, 80 ; 84 ; 88) étant aligné avec un axe longitudinal (17) dudit réceptacle de tige de soupape (15), et
au moins une ouverture d'entrée d'air (20) étant disposée dans une enveloppe extérieure du logement de manière à être espacée de ladite ouverture (21) pour recevoir ladite cartouche (2) et de ladite ouverture d'embout buccal (22), ladite au moins une ouverture d'entrée d'air (20) étant en communication fluidique avec ladite ouverture d'embout buccal (22), et
un axe longitudinal (25) de la partie d'embout buccal (13) étant disposé selon un angle et non parallèlement par rapport ledit axe longitudinal (18) de ladite orifice (16 ; 73, 74 ; 76, 77 ; 79, 80 ; 84 ; 88),
**caractérisé en ce qu'**une ouverture d'entrée d'air (20) de ladite au moins une ouverture d'entrée d'air (20) est disposée pour produire un courant d'air essentiellement perpendiculaire à la direction du nuage d'aérosol et est positionnée sur une ligne de vision (29) qui passe à travers ladite ouverture d'embout buccal (22) et est alignée avec ledit axe longitudinal (25) de la partie d'embout buccal (13), et est disposée pour produire un courant d'air essentiellement perpendiculaire à la direction du nuage d'aérosol.

2. Actionneur selon la revendication 1, dans lequel ladite au moins une ouverture d'entrée d'air (20) est disposée dans une partie de ladite enveloppe extérieure dudit logement s'étendant depuis ledit élément (14) en direction de ladite ouverture d'embout buccal (22).

3. Actionneur selon la revendication 1 ou la revendication 2, dans lequel ledit logement comprend une paroi, qui est orientée selon un angle par rapport ledit axe longitudinal (25) de ladite partie d'embout buccal (13), une ouverture d'entrée d'air (20) de ladite au moins une ouverture d'entrée d'air (20) est disposée dans ladite paroi.

4. Actionneur selon l'une quelconque des revendications précédentes, dans lequel chaque ouverture d'entrées d'air (20) de ladite au moins une ouverture d'entrée d'air (20) est respectivement positionnée sur une ligne de vision (29) qui passe à travers ladite ouverture d'embout buccal (22) et est alignée avec ledit axe longitudinal (25) de la partie d'embout buccal (13) .

5. Actionneur selon l'une quelconque des revendications précédentes, dans lequel ledit élément (14) et ladite ouverture d'entrée d'air (20) sont configurés de telle sorte, que lors de l'utilisation de l'actionneur (11 ; 31 ; 41 ; 51 ; 91) tout l'air sortant par ladite ouverture d'embout buccal (22) est aspiré dans un intérieur dudit logement à travers ladite au moins une ouverture d'entrée d'air (20).

6. Actionneur selon l'une quelconque des revendications précédentes, dans lequel ledit élément (14) s'étant sur une zone en coupe de ladite partie réceptrice du cartouche (12).

7. Actionneur selon la revendication 6, dans lequel ledit élément (14) est configuré pour bloquer le passage de gaz au-delà dudit élément (14) radialement vers l'extérieur dudit orifice (16 ; 73, 74 ; 76, 77 ; 79, 80 ; 84 ; 88).

8. Actionneur selon l'une quelconque des revendications précédentes, dans lequel ledit orifice (73, 74 ; 76, 77 ; 79, 80) a au moins une partie (73 ; 76 ; 79) effilée vers ladite face (19) dudit élément (14) a l'opposé du réceptacle de tige de soupape (15).

9. Actionneur selon la revendication 8, dans lequel la partie effilée (79) dudit orifice (79, 80) a un diamètre maximum correspondant à un diamètre extérieur de ladite tige de soupape (3) .

10. Actionneur selon la revendication 8, dans lequel la partie effilée (76) dudit orifice (76, 77) a un diamètre maximum correspondant à un diamètre interne de ladite tige de soupape (3) .

11. Actionneur selon l'une quelconque des revendications précédentes, dans lequel une chambre de dilatation (82, 83 ; 86, 87) est formée dans ledit élément (14), ladite chambre de dilatation (82, 83 ; 86, 87) est en communication fluidique avec ledit orifice (84 ; 88) et ledit réceptacle de tige de soupape (15) et a un axe longitudinal aligné avec ledit axe longitudinal (17) dudit réceptacle de tige de soupape (15).

12. Actionneur selon l'une quelconque des revendications précédentes, dans lequel ledit axe (18) dudit orifice (16 ; 73, 74 ; 76, 77 ; 79, 80 ; 84) est disposé selon un angle (28) supérieur à 90° par rapport audit axe longitudinal (25) de la partie d'embout buccal (13).

13. Inhalateur-doseur, comprenant l'actionneur (11 ; 31 ; 41 ; 51 ; 91) selon l'une quelconque des revendications précédentes et une cartouche (2) munie d'une valve doseuse (32) qui comprend une tige de soupape (3) pour être adaptée dans ledit réceptacle de tige de soupape (15) formé dans ledit élément (14) dudit actionneur (11 ; 31 ; 41 ; 51), ladite cartouche (2) contenant une formulation d'aérosol.

14. Utilisation non-thérapeutique d'un actionneur (11 ; 31 ; 41 ; 51 ; 91) selon l'une quelconque des revendications 1 à 12 pour la distribution d'une formulation d'un aérosol à partir d'une cartouche (2).

Fig. 1

Fig. 2

Fig. 3

Fig. 4

Fig. 5

Fig. 6

Fig. 7

Fig. 8

Fig. 9

Fig. 10A

Fig. 10B

Fig. 11A

Fig. 11B

Fig. 12

Fig. 13

Fig. 14

Fig. 15

Fig. 16

121

122

123

124

I

II

III

IV

Fig. 17

128

94

92

125

126

125

127

Fig. 18A

Fig. 18B

Fig. 19

Fig. 20A

Fig. 20B

Fig. 20C

Fig. 21A

Fig. 21B

Fig. 21C

Fig. 22A

Fig. 22B

Fig. 23

Fig. 24

Fig. 25

Fig. 26

Fig. 27A

Fig. 27B

Fig. 28

Fig. 29

Fig. 30

Fig. 31

Fig. 32

Fig. 33

Fig. 34

Fig. 35

Fig. 36

Fig. 37

Fig. 38

Fig. 39

Fig. 40

**EP 2 613 828 B1**

REFERENCES CITED IN THE DESCRIPTION

**Patent documents cited in the description**

- WO 2009003657 A1
- US 4972830 A
- US 5435297 A
- US 3001524 A
- GB 2104393 A
- US 6062214 A
- US 5669376 A
- US 4796614 A